# EUROPEAN PATENT SPECIFICATION

(11) **EP 3 500 562 B1**
(45) Date of publication and mention of the grant of the patent: **15.11.2023**
(21) Application number: 17849203.9
(22) Date of filing: 18.08.2017
(51) Int. Cl.: C07D 401/04, C07D 413/04, A61K 35/28, A61K 35/51, C12N 5/0789, A61P 7/06, A61P 37/00, A61K 35/00

(54) **SUBSTITUTED AZOLE DERIVATIVES FOR GENERATION, PROLIFERATION AND DIFFERENTIATION OF HEMATOPOIETIC STEM AND PROGENITOR CELLS**
SUBSTITUIERTE AZOLDERIVATE ZUR ERZEUGUNG, PROLIFERATION UND DIFFERENZIERUNG VON HÄMATOPOIETISCHEN STAMM- UND VORLÄUFERZELLEN
DÉRIVÉS D'AZOLE SUBSTITUÉS POUR LA GÉNÉRATION, LA PROLIFÉRATION ET LA DIFFÉRENCIATION DE CELLULES SOUCHES ET PROGÉNITRICES HÉMATOPOÏÉTIQUES

(30) Priority: 18.08.2016 SG 10201606886V
(43) Date of publication of application: 26.06.2019
(73) Proprietor: National University of Singapore, Singapore 119077 (SG); Singapore Health Services Pte. Ltd., Singapore 168753 (SG)
(72) Inventor: BARI, Sudipto, Singapore 119077 (SG); CHAI, Christina, Li, Lin, Singapore 119077 (SG); CHIU, Gigi, Ngar, Chee, Singapore 119077 (SG); HWANG, William, Ying, Khee, Singapore 119077 (SG); LOW, Joo, Leng, Singapore 119077 (SG); ZHONG, Qixing, Singapore 119077 (SG)
(74) Representative: Marks & Clerk LLP
(86) International application number: PCT/SG2017/050409
(87) International publication number: WO 2018/048346

(56) References cited:
- WO-A1-2013/059357
- WO-A1-2013/086029
- WO-A1-2013/086436
- WO-A1-2015/119579
- WO-A1-2015/148716
- WO-A1-2015/161373
- WO-A1-2017/075274
- WO-A2-2010/059401
- WO-A2-2012/141971
- CN-A- 104 144 931
- CN-A- 105 713 879
- XIUBO FAN ET AL: "Low-dose insulin-like growth factor binding proteins 1 and 2 and angiopoietin-like protein 3 coordinately stimulate ex vivo expansion of human umbilical cord blood hematopoietic stem cells as assayed in NOD/SCID gamma null mice", STEM CELL RESEARCH & THERAPY, BIOMED CENTRAL LTD, LONDON, UK, vol. 5, no. 3, 30 May 2014 (2014-05-30), page 71, XP021189859, ISSN: 1757-6512, DOI: 10.1186/SCRT460
- Sudipto Bari ET AL: "NANOMATERIAL AND SMALL MOLECULE APPROACHES IN HUMAN HEMATOPOIETIC STEM AND PROGENITOR CELL EXPANSION - A THESIS SUBMITTED FOR THE DEGREE OF DOCTOR OF PHILOSOPHY DEPARTMENT OF PHARMACY FACULTY OF SCIENCE NATIONAL UNIVERSITY OF SINGAPORE 2017", , 17 January 2017 (2017-01-17), XP055664762, Retrieved from the Internet: URL:https://scholarbank.nus.edu.sg/handle/ 10635/135723 [retrieved on 2020-02-04]
- SUDIPTO BARI ET AL: "Ex Vivo Expansion of CD34 + CD90 + CD49f + Hematopoietic Stem and Progenitor Cells from Non-Enriched Umbilical Cord Blood with Azole Compounds : Expansion of UCB HSPC from MNC with Azole Compound", STEM CELLS TRANSLATIONAL MEDICINE, vol. 7, no. 5, 2 February 2018 (2018-02-02), pages 376-393, XP055664746, US ISSN: 2157-6564, DOI: 10.1002/sctm.17-0251
- BARI S. et al.: "Expansion and Homing of Umbilical Cord Blood Hematopoietic Stem and Progenitor Cells for Clinical Transplantation", Biol Blood Marrow Transplant, vol. 21, no. 6 30 December 2014 (2014-12-30), pages 1008-1019, XP029155489, [retrieved on 2017-10-20]
- WANG Y. et al.: "Inhibition of p38 Mitogen-Activated Protein Kinase Promotes Ex Vivo Hematopoietic Stem Cell Expansion", Stem Cells And Development, vol. 20, no. 7 3 January 2011 (2011-01-03), pages 1143-1152, XP055446478, [retrieved on 2017-10-20]
- L I X. et al.: "Inhibition of Both Activated p38 MAPK and mTOR C1 Potentiates the Effect of SR1 on Promotion of Hematopoietic Stem Cell Expansion Ex Vivo", Blood, vol. 126, no. 23 3 December 2015 (2015-12-03), XP035051060, [retrieved on 2017-10-20]
- BARI S. et al.: "Small Molecule Based Ex Vivo Expansion of CD 34+ CD 90+ CD 49f+ Hematopoietic Stem & Progenitor Cells from Non-Enriched Umbilical Cord Blood Mononucleated Cells", Blood, vol. 128, no. 22 1 December 2016 (2016-12-01), page 2321, XP009513924, ISSN: 0006-4971 Retrieved from the Internet: URL:http://www.bloodjournal.org/content/12 8/22/2321?sso-checked=true [retrieved on 2017-10-20]

## Description

### FIELD OF THE INVENTION

This invention is related to substituted azole derivatives and their use in ex vivo expansion of CD34 expressing hematopoietic stem and progenitor cells (HSPC) in a biological sample; more particularly the expansion of these cells obtained from non-enriched, i.e., the mononuclear fraction of the biological sample. This invention further describes the transplantation regimen of the expanded hematopoietic graft developed through xenotransplantation studies.

### BACKGROUND OF THE INVENTION

Hematopoietic stem cell transplants (HSCT) are used to correct defects in blood cells that lead to malignant and benign disorders by replacing the diseased ones with healthy donor cells [Gratwohl A, et al., JAMA 303(16): 1617-1624 (2010)]. To date over one million HSCT have been performed with mobilized peripheral blood stem cells (PBSC), bone marrow (BM) and umbilical cord blood (UCB) being the sources of graft. In the past decade, the number of registry HSCT has gone up by three times primarily to treat malignant blood disorders like acute myeloid leukemia (National Marrow Donor Programme, USA) [Lund TC, et al., Nature reviews. Clinical Oncology 12(3):163-74 (2015)]. Irrespective of graft source, about 6,500 transplants were performed worldwide in 2014. Although PBSC or BM is still considered to be the main source of graft, UCB emerged to be an effective alternative for about 31 % of patients who underwent HSCT in 2014 [Bari S, et al., Biol Blood Marrow Transplant 21(6):1008-19 (2015)].

Since the first UCB transplant was performed in 1988, to treat successfully a Fanconi's anemia patient, this biological waste has been actively stored in public and private blood banks and has recently been approved by the Food and Drug Administration (FDA, USA) as a legitimate source of HSPC [Gluckman E, et al., Nouv Rev Fr Hematol 32(6):423-425 (1990); Voelker R. JAMA 306(22): 2442 (2011)]. Compared to BM or PBSC, UCB transplantations (UCBT) are associated with a greater ease of HSPC collection, prompt availability (>700,000 registry UCB units stored worldwide), lower risk of infectious disease transmission, greater tolerance across human leukocyte antigen (HLA) barriers and a lower incidence of graft-vs-host-disease (GVHD) [Lund TC, et al., Nature reviews. Clinical Oncology 12(3): 163-74 (2015); Bari S, et al., Biol Blood Marrow Transplant 21(6): 1008-19 (2015)]. Also, in several meta-analyses, UCBT has been shown to lead to equivalent outcomes to fully matched BM transplants in both adult and pediatric patients lacking matched sibling donors [Hwang WYK, et al., Biol Blood Marrow Transplant 13(4): 444-453 (2007)]. Worldwide, approximately 40% of Caucasians, and up to 55-80% of non-Caucasian patients will not be able to find an 8/8 HLA-A,-B,-C, and -DR matched unrelated donor (MUD), which means over 6,000 patients per year are eligible for UCBT [Cunha R, et al., Bone Marrow Transplant 49(1): 24-29 (2014); Barker JN, etal., Biol Blood Marrow Transplant 16(11): 1541-1548 (2010)]. However, in 2014, only 960 UCBT (NMDP, USA) were performed, primarily due to the problem of low total nucleated cell (TNC) dosage associated with banked UCB grafts, which immensely limits their clinical usage.

Although UCBT have been used successfully in pediatric patients, where a single graft is able to fulfill the minimum clinical dose of 25 million cells/kg of body weight, there are significant challenges to their use in adult patients [Gluckman E, Rocha V. Cytotherapy 7(3): 219-227 (2005)]. The characteristically slower rate of hematopoietic recovery after UCBT in adults, relative to BM or PBSC, is a consequence of a lower TNC and HSPC content for mediating successful transplantation, as well as an intrinsic cellular deficiency for functions related to engraftment in UCB grafts [Ballen KK, et al., Blood 122(4): 491-498 (2013)]. Median neutrophil engraftment times, which are early measures of the success of a transplant, are typically more than 25 days for unmanipulated UCB grafts versus a median of approximately 14 days and 18 days, respectively, for PBSC or BM grafts [Lund TC, et al., Nature reviews. Clinical Oncology 12(3): 163-74 (2015)]. Reconstitution times for other immune cells such as T, B and NK cells, which typically occurs later (>3 months) than neutrophil and platelet recovery, are delayed more significantly after UCBT due to the relatively immature immune status of UCB cells [Komanduri KV, et al., Blood 110(13): 4543-4551 (2007)]. The profound delay in hematopoietic reconstitution increases risk of opportunistic microbial and viral infection in the pancytopenic recipients thus contributing to the high transplant related mortality (TRM) of >30% following UCBT [Bari S, et al., Biol Blood Marrow Transplant 21(6): 1008-19 (2015); Hofmeister CC, et al., Bone Marrow Transplant 39(1): 11-23 (2007)]. However, the infection and mortality risks appear to be lower with a higher infused cell dose for transplantation [Kelly SS, et al., Bone Marrow Transplant 44(10): 673-681 (2009); Dahlberg A, et al., Blood 117(23): 6083-6090 (2011)]. Several groups have attempted to expand the stem cells component of adult tissues. Mouse muscle stem cell proliferation was enhanced by contact with an inhibitor of the p38 MAPK signaling pathway, such as the azole-based small molecule SB203580 [WO 2012/141971 A2]. Similarly, MAPK inhibitors have been used to enhance engraftment of human hematopoietic stem cells [WO 2017/075274 A1]. Retinoic acid signaling inhibitors such as DEAB, optionally with p38 MAPK inhibitors such as ralimetinib (e.g., LY222882O dimesylate), have been used to differentiate ES cells and PSC [WO 2013/086029 A1]. Hematopoietic stem cells have been expanded ex vivo in the presence of cytokines such as stem cell factor (SCF), thrombopoietin (TPO), FLT3 ligand and IGF binding proteins [Fan X, et al., Stem Cell Research & Therapy 5(3): 71ff (2014)]. Given the advantages of UCBT, it is desirable to enable UCB to be a graft of primary choice for HSCT. To realize this objective, however, it is necessary to increase the number of TNC and HSPC prior to transplanting in adults who have received the appropriate preparative regimen (myeloablative or reduced intensity conditioning).

Accordingly, there is a need to provide a more abundant supply of TNC and HSPC for grafting, and a method for producing same.

### SUMMARY OF THE INVENTION

We describe a method of expanding phenotypically and functionally defined HSPC from frozen thawed UCB-mononuclear cells (MNC) using an azole-based small molecule, IM-29, and derivatives thereof. Phenotypically and functionally defined HSPC may also be expanded in bone marrow and/or mobilized peripheral blood samples using the small molecules of the invention. If so desired, the small molecules of the invention could be used to expand an enriched CD34+ HSPC cohort of cells from a UCB, bone marrow or mobilized peripheral blood sample.

According to a preferred aspect, the present invention provides a method for *ex vivo* expansion of the total nucleated cells and/or a subset of CD45+CD34+ hematopoietic stem cells and progenitor cells component of an umbilical cord blood, bone marrow or mobilized peripheral blood sample comprising the steps of:
(i) culturing a total nucleated cells or a mononucleated cell fraction or CD45+CD34+ hematopoietic stem cells and progenitor cells component of the sample in media; and
(ii) contacting the cell(s) of step (i) with a composition comprising at least one azole-based small molecule, wherein the at least one azole-based small molecule is represented by formula (I), 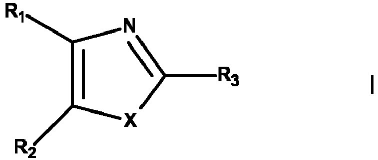
wherein:
X represents NR₄ or O, where R₄ is H or methyl;
R₁ represents phenyl or pyridinyl (which are unsubstituted or substituted with one or more substituents selected from Cl, Br, F and methyl, (which latter group is unsubstituted or substituted with F));
R₂ represents phenyl, pyridyl or dihydropyranyl (which are unsubstituted or substituted with one or more substituents selected from Cl, Br, F and methyl (which latter group is unsubstituted or substituted with F));
R₃ represents H, or naphthyl which is unsubstituted or substituted with one or more groups selected from CI, F and OR₅ where R₅ is H or methyl, or;
salts and solvates thereof.

The at least one azole-based small molecule may be selected from:
(A) the list:
   (i) 4-[2-(1-fluoronaphthalen-2-yl)-4(5)-(4-fluorophenyl)-1*H*-imidazol-5(4)-yl]pyridine;
   (ii) 4-[2-(1-fluoronaphthalen-2-yl)-4-(*m*-tolyl)-1*H*-imidazol-5-yl]pyridine;
   (iii) 4-[2-(naphthalen-2-yl)-4(5)-(*m*-tolyl)-1*H*-imidazol-5(4)-yl]pyridine;
   (iv) 4-[2-(naphthalen-2-yl)-4(5)-(4-fluorophenyl)-1*H*-imidazol-5(4)-yl]pyridine;
   (v) 4-[2-(1-bromonaphthalen-2-yl)-4(5)-(4-fluorophenyl)-1*H*-imidazol-5(4)-yl]pyridine;
   (vi) 4-[2-(1-fluoronaphthalen-2-yl)-4-[3-(trifluoromethyl)phenyl]-1*H-*imidazol-5-yl]pyridine;
   (vii) 2-(1-fluoronaphthalen-2-yl)-4-(pyridin-4-yl)-5-(*m*-tolyl)oxazole;
   (viii) 5(4)-(3,6-dihydro-2*H*-pyran-4-yl)-2-(1-fluoronaphthalen-2-yl)-4(5)-(m-tolyl)-1*H*-imidazole;
   (ix) 5(4)-(3,6-dihydro-2*H*-pyran-4-yl)-2-(6-methoxynaphthalen-2-yl)-4(5)-(*m*-tolyl)-1*H*-imidazole;
   (x) 5(4)-(3,6-dihydro-2*H*-pyran-4-yl)-2-(1-fluoronaphthalen-2-yl)-4(5)-(4-fluorophenyl)-1*H*-imidazole;
   (xi) 4-(4(5)-(4-fluorophenyl)-2-(7-methoxynaphthalen-2-yl)-1*H*-imidazol-5(4)-yl)pyridine;
   (xii) 4-[4(5)-(*m*-tolyl)-1*H*-imidazol-5(4)-yl]pyridine; and
   (xiii) 4-[4(5)-(4-fluorophenyl)-1*H*-imidazol-5(4)-yl]pyridine; or
(B) the list:
   (i) 4-[2-(1-fluoronaphthalen-2-yl)-4(5)-(4-fluorophenyl)-1*H*-imidazol-5(4)-yl]pyridine;
   (ii) 4-[2-(1-fluoronaphthalen-2-yl)-4-(*m*-tolyl)-1*H*-imidazol-5-yl]pyridine;
   (iii) 4-[2-(naphthalen-2-yl)-4(5)-(*m*-tolyl)-1H-imidazol-5(4)-yl]pyridine;
   (iv) 4-[2-(naphthalen-2-yl)-4(5)-(4-fluorophenyl)-1*H*-imidazol-5(4)-yl]pyridine;
   (v) 4-[2-(1-bromonaphthalen-2-yl)-4(5)-(4-fluorophenyl)-1*H*-imidazol-5(4)-yl]pyridine;
   (vi) 4-[2-(1-fluoronaphthalen-2-yl)-4-[3-(trifluoromethyl)phenyl]-1*H-*imidazol-5-yl]pyridine; and
   (vii) 2-(1-fluoronaphthalen-2-yl)-4-(pyridin-4-yl)-5-(*m*-tolyl)oxazole.

The hematopoietic stem cells and progenitor cells may be expanded in the presence of at least one cytokine selected from the group comprising stem cell factor (SCF), thrombopoietin (TPO), Fms-related tyrosine kinase 3 ligand (FLT-3L) and insulin-like growth factor binding protein 2 (IGFBP-2). Preferably, the hematopoietic stem cells and progenitor cells are expanded in the presence of at least two, at least three or all four of SCF, TPO, FLT-3L and IGFBP-2. More preferably, the hematopoietic stem cells and progenitor cells are expanded in the presence of 100 ng/ml SCF, 100 ng/ml TPO, 50ng/ml FLT-3L and 20 ng/ml IGFBP-2.

In another preferred embodiment of the invention, the method comprises culturing the umbilical cord blood mononuclear cell(s) with the at least one azole-based small molecule for a period of at least 9 days.

In another preferred embodiment of the invention, the cytokines are added to the culture at day 0 and/or at day 7.

In another preferred embodiment of the invention, the at least one azole-based small molecule is added to the culture at day 0 and/or at day 7.

In another preferred embodiment of the invention, the method further comprises the step of harvesting the cells after about 10 or 11 days when optimal expansion is observed.

In another preferred embodiment of the invention, CD45+CD34+CD38-CD45RA-hematopoietic progenitor cells are expanded.

In another preferred embodiment of the invention, CD45+CD34+CD38-CD45RA-CD90+ (HSC1) hematopoietic stem cells are expanded.

In another preferred embodiment of the invention, CD45+CD34+CD38-CD45RA-CD90+CD49f+ (HSC2) hematopoietic stem cells are expanded.

In another preferred embodiment of the invention, the expanded hematopoietic stem and progenitor cells possess normal karyotype and do not exhibit any leukemic transformation.

In another aspect of the invention, there is provided a composition comprising at least one azole-based small molecule defined according to any aspect of the invention; and at least one cytokine selected from the group comprising SCF, TPO, FLT-3L and IGFBP-2 for use in *ex vivo* expansion of the hematopoietic stem cells and progenitor cells component of umbilical cord blood, bone marrow and/or mobilized peripheral blood.

In a preferred embodiment of the invention, the at least one azole-based small molecule is used in *ex vivo* expansion of the hematopoietic stem cells and progenitor cells component of umbilical cord blood.

In another aspect of the invention, there is provided a use of a composition comprising at least one azole-based small molecule as herein defined, in *ex vivo* expansion of the hematopoietic stem cells and progenitor cells component of umbilical cord blood, bone marrow and/or mobilized peripheral blood. Preferably, the hematopoietic stem cells and progenitor cells component is from umbilical cord blood.

### BRIEF DESCRIPTION OF THE FIGURES

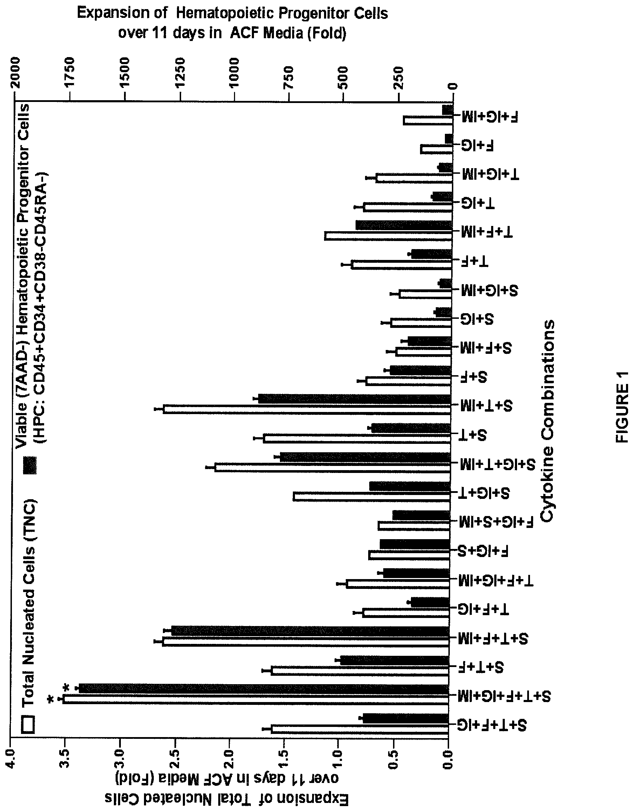

**Figure 1** shows fold expansion of viable (7AAD-) hematopoietic progenitor cells (HPC: CD45+CD34+CD38-CD45RA-) and total nucleated cells (TNC) in cultures that lasted for 11 days with animal component free (ACF) media, different combinations of cytokines with and without IM-29. Media, cytokines and IM-29 were replenished at day 7. The concentrations of each cytokine are as follows: S represents SCF at 100 ng/ml; T represents TPO at 100 ng/ml; F represents FLT-3L at 50 ng/ml; and IG represents IGFBP-2 at 20 ng/ml. The small molecule IM-29 is represented by IM and administered at a concentration of 5.0 µM. *P < 0.05 compared to respective groups in all other conditions. Data represents mean ± SD for n = 3.

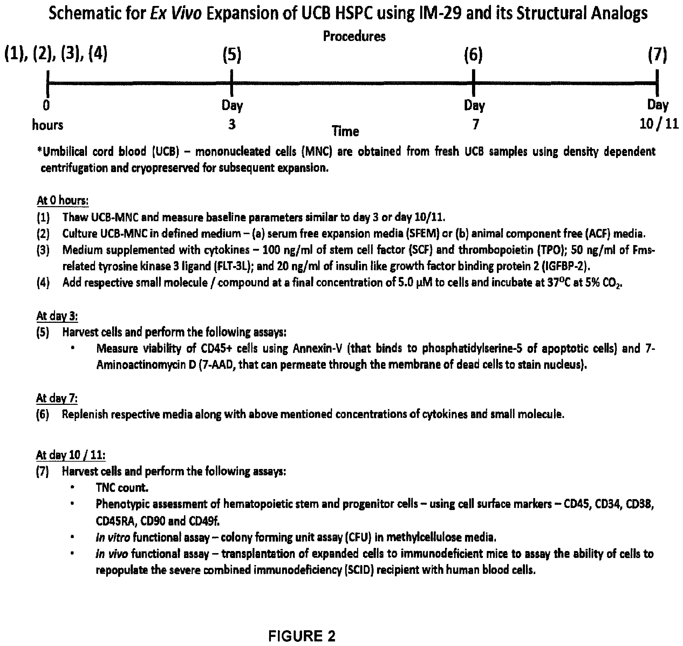

**Figure 2** shows a schematic describing the method that enables *ex vivo* expansion of UCB HSPC using IM-29 and its structural analogues.

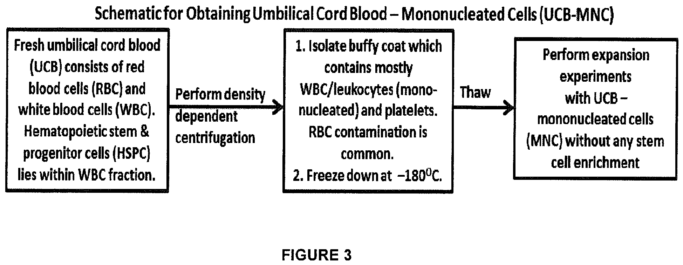

**Figure 3** is a schematic diagram describing the process of obtaining mononucleated cells (MNC) from fresh UCB.

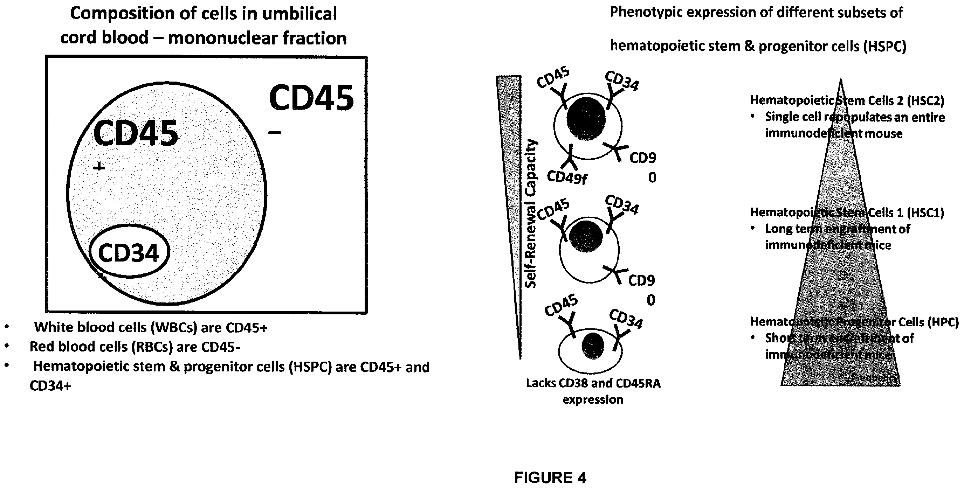

**Figure 4** is a schematic depiction of the composition of cells in UCB-MNC fraction and phenotypic expression of different subsets of HSPC.

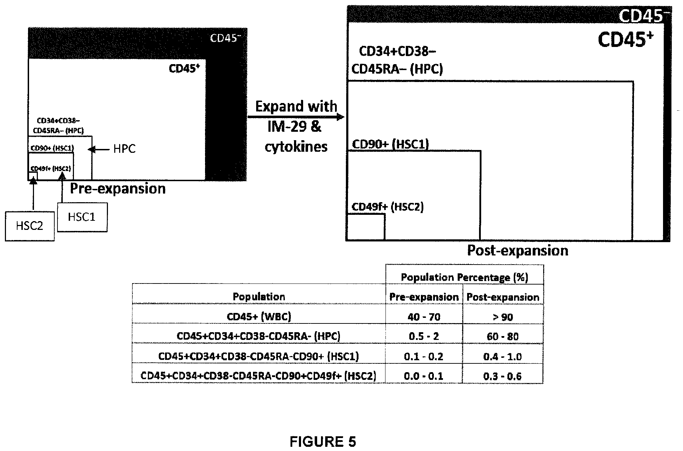

**Figure 5** is a schematic diagram describing the change in proportion of cells in UCB graft due to *ex vivo* expansion with IM-29 using mononucleated cells.

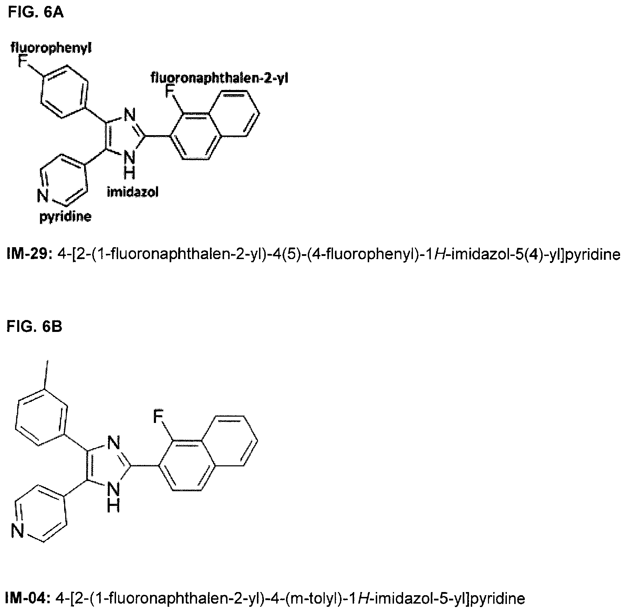

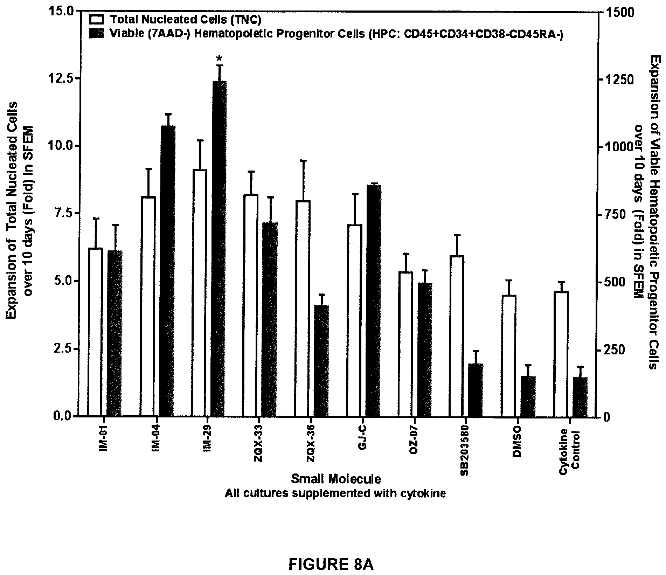

**Figure 6A** shows that the small molecule IM-29 (molecular weight, MW: 383.12 g/mol) gives optimal expansion of UCB (>1200-fold increase of viable hematopoietic progenitor cells, HPC, defined by phenotypic expression of CD45+CD34+CD38-CD45RA-, shown in Figure 8 (A)).
**Figure 6B** shows the structure of small molecule IM-04 (MW: 379.43 g/mol) which can effect a 1000 to 1150-fold increase of viable hematopoietic progenitor cells defined by phenotypic expression of CD45+CD34+CD38-CD45RA-, shown in Figure 8 (A).

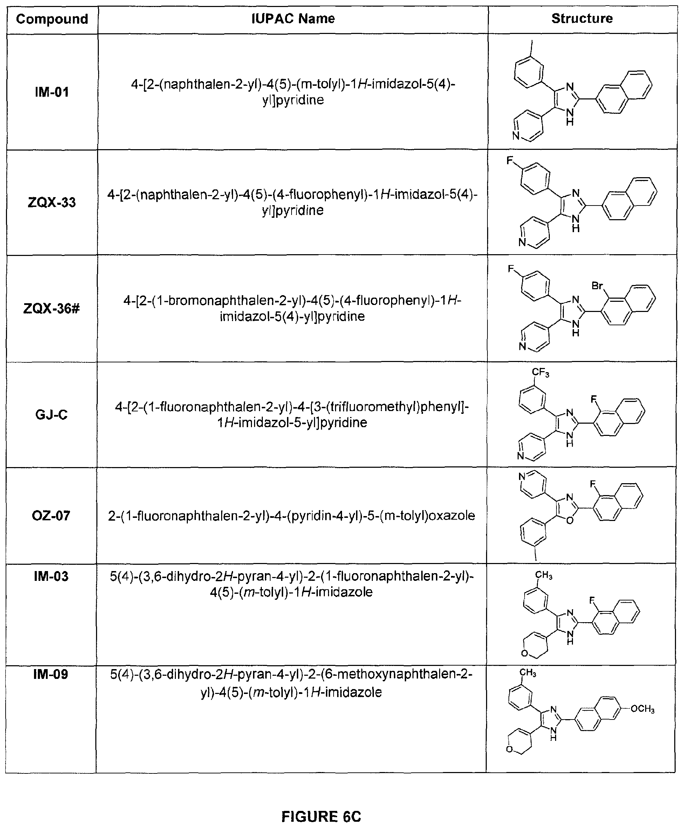

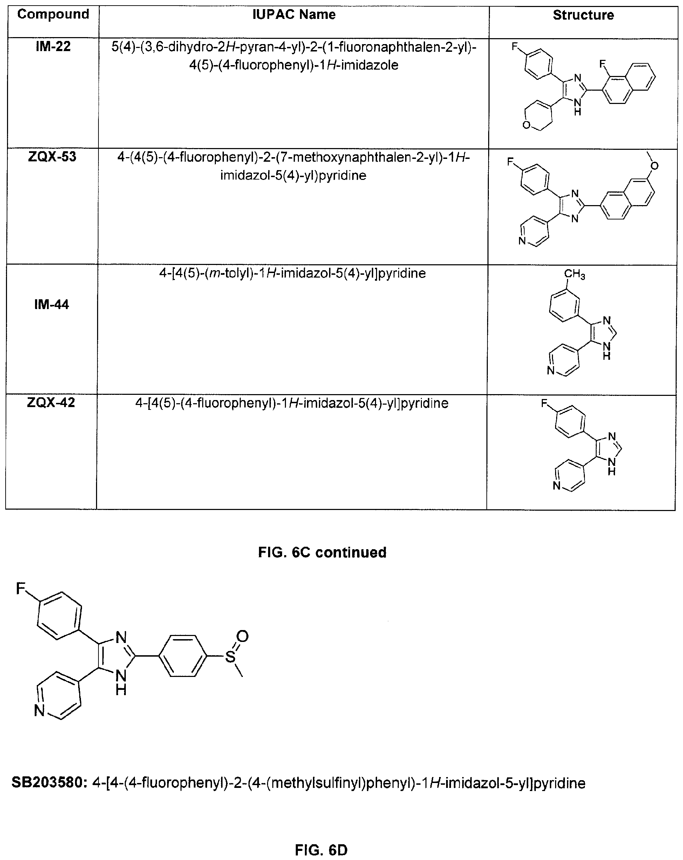

**Figure 6C** shows the structure of small molecules IM-01 (MW: 361.45 g/mol), ZQX-33 (MW: 365.13 g/mol), ZQX-36 (MW: 443.04 g/mol), GJ-C (MW:433.41 g/mol), OZ-07 (MW: 380.42 g/mol), IM-03 (MW: 384.16 g/mol), IM-09 (MW: 396.18 g/mol), IM-22 (MW: 388.14 g/mol), ZQX-53 (MW: 394.14 g/mol), IM-44 (MW: 235.11 g/mol) and ZQX-42 (MW: 239.09 g/mol) which are structural analogues of IM-29 (Figure 6 (A)) and IM-04 (Figure 6 (B)) which gave 400 to 900-fold increase of viable hematopoietic progenitor cells (HPC) defined by phenotypic expression of CD45+CD34+CD38-CD45RA- (shown in Figure 8 (A).
**Figure 6D** shows the structure of parent compound SB203580 (MW: 377.43 g/mol) which is an established p38 mitogen-activated protein kinases (MAPK) inhibitor. The optimal working concentration of SB203580 is known to be 5.0 µM.

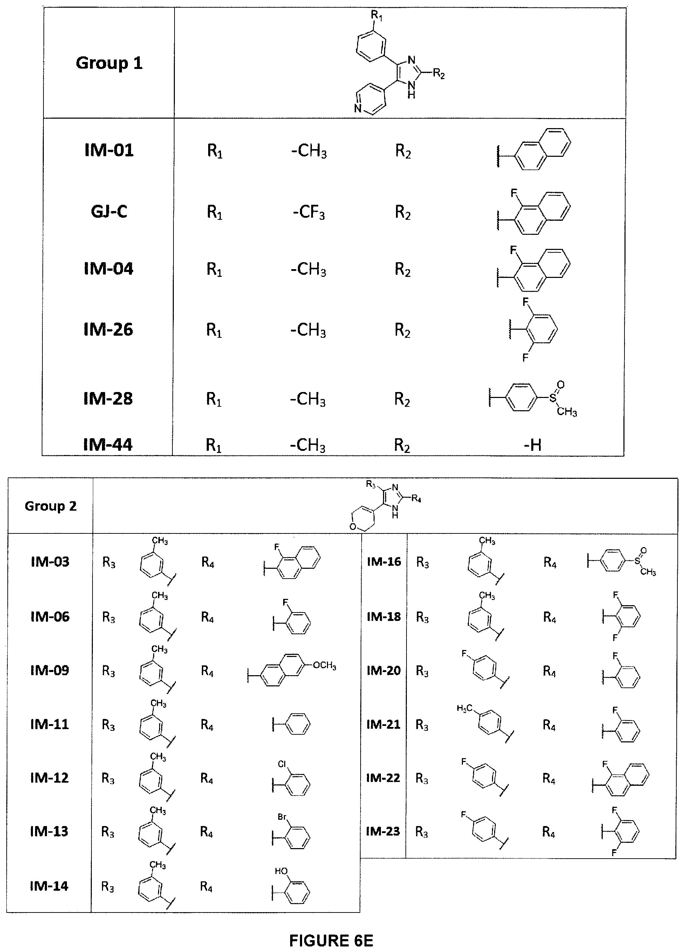

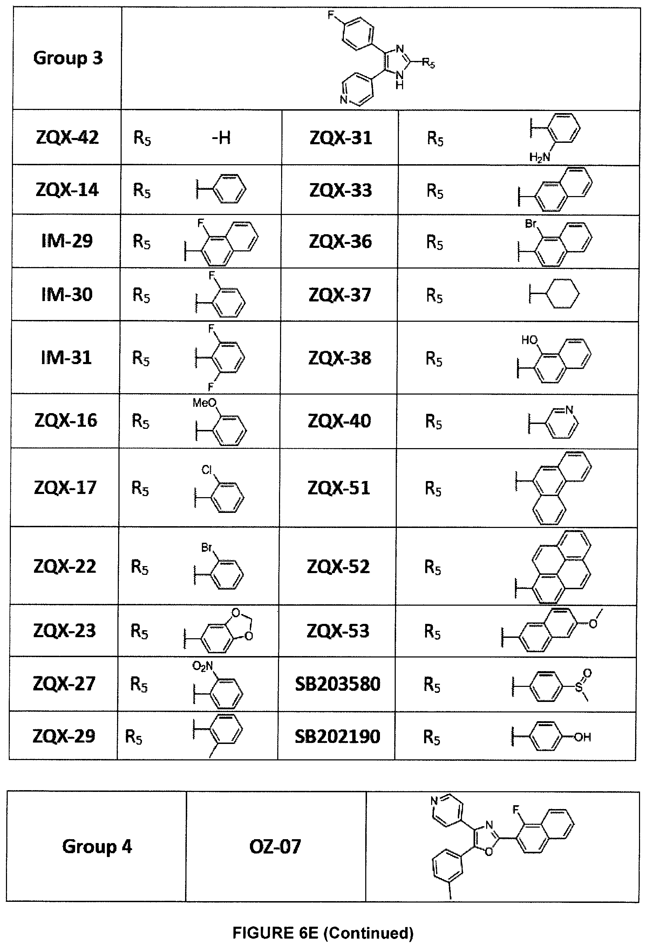

**Figure 6E** shows structures of the analogues of the parent compound SB203580 that were generated to study the expansion of hematopoietic stem and progenitor cell (HSPC) from umbilical cord blood (UCB) mono-nucleated cells (MNC). Based on the structural and chemical modification, the generated analogues were subdivided into four broad groups 1-4.

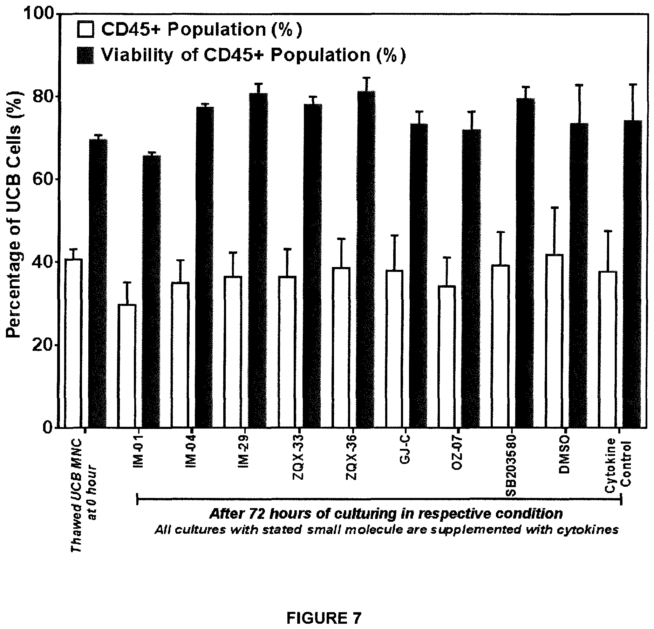

**Figure 7** shows the effect of IM-29 and its structural analogues at 5.0 µM on the CD45+ population and cell viability at 72 hours using frozen-thawed MNC from three different UCB samples. SB203580, DMSO and cytokines alone in serum-free expansion media (SFEM) served as the reference compound, vehicle and blank control, respectively. Data represents mean ± SEM for n = 3. The effect of IM-29 and its structural analogues on UCB HSPC was assessed using a viability assay that includes staining the UCB cells with Annexin-V (binds to phosphatidylserine 5 expressed on early apoptotic cells) and 7-aminoactinomycin D (7-AAD that stains dead cells). This flow cytometer-based assay was chosen since published reports [Bari S, et al., Biol Blood Marrow Transplant 21(6): 1008-19 (2015)] suggest that spontaneous induction of apoptosis of CD45+ leukocytes (HSPC is a subset of this population) is a prominent problem of HSPC expansion cultures. This data suggests that neither of the small molecules has acute toxicity to the UCB-MNC cells.
**Figure 8A** shows the fold expansion of viable (7AAD-) hematopoietic progenitor cells (HPC: CD45+CD34+CD38-CD45RA-) and total nucleated cells (TNC) in cultures that lasted for 10 days with serum-free expansion media (SFEM), cytokine and respective small molecule being replenished at day 7. **P* < 0.01 compared to all other conditions in respective group. SB203580, DMSO and cytokines alone in SFEM served as the reference compound, vehicle and blank control, respectively. Data represents mean ± SEM for n = 4.

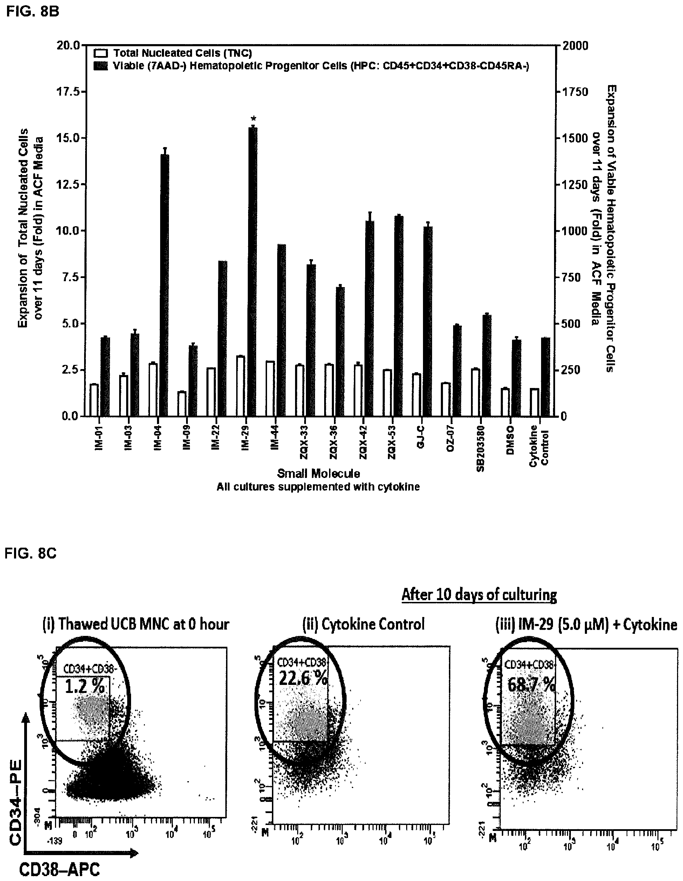

**Figure 8B** shows the fold expansion of viable (7AAD-) hematopoietic progenitor cells (HPC: CD45+CD34+CD38-CD45RA-) and total nucleated cells (TNC) in cultures that lasted for 11 days with animal-component-free media (ACF), cytokine and respective small molecule being replenished at day 7. **P* < 0.01 compared to all other conditions in respective group. SB203580, DMSO and cytokines alone in ACF media served as the reference compound, vehicle and blank control, respectively. Data represents mean ± SEM for n = 3.
**Figure 8C** shows representative dot plots from flow cytometric analysis depicting CD34+CD38- population which is a subset of the CD45+ cells of (i) thawed UCB MNC at 0 hours followed by culturing for 10 days in (ii) cytokine control and (iii) 5.0 µM of IM-29 supplemented with cytokines using serum-free expansion media (SFEM).

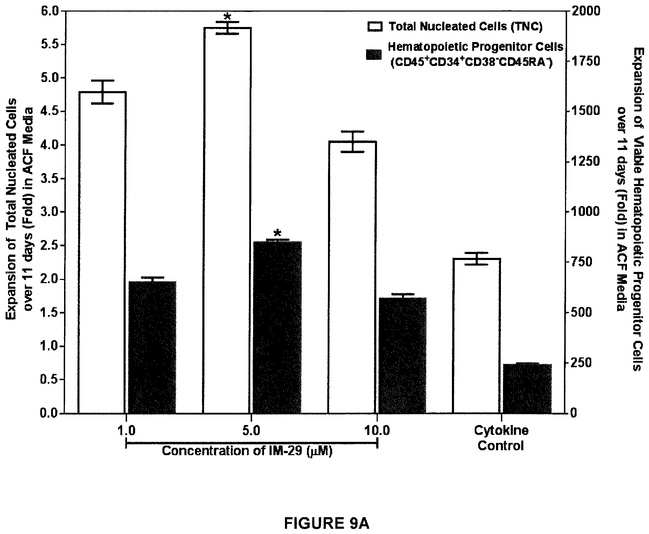

**Figure 9A** shows the fold expansion of viable (7AAD-) hematopoietic progenitor cells (HPC: CD45+CD34+CD38-CD45RA-) and total nucleated cells (TNC) in cultures that lasted for 11 days with animal-component-free media (ACF), cytokine and respective small molecule being replenished at day 7. The concentrations of IM-29 used are 1.0, 5.0 and 10.0 µM. Cytokines alone in ACF media served as the blank control. **P* < 0.01 compared to respective group. Data represents mean ± SD for n = 3.

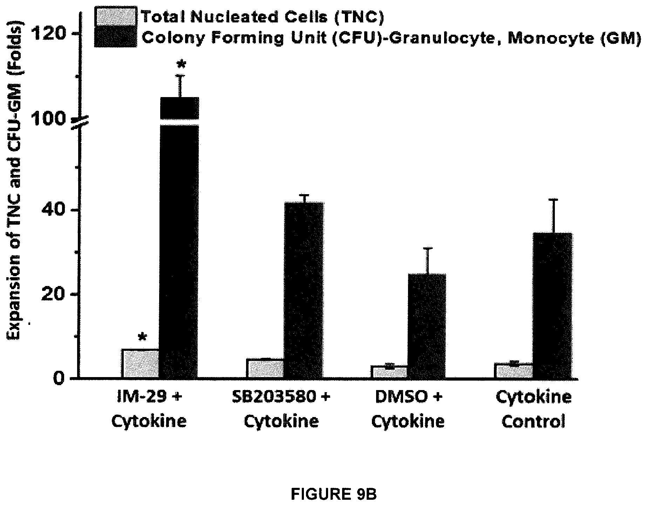

**Figure 9B** shows *ex vivo* expansion of total nucleated cells (TNC) and colony forming unit (CFU) - granulocyte, macrophage (GM) when two separate UCB units without pre-selection of stem cells were cultured in 5.0 µM of IM-29 and basal cytokines. The expansion cultures lasted for 10 days with SFEM, cytokine and IM-29 replenishment being done on day 7. SB203580, DMSO and cytokines alone in SFEM served as the reference compound, vehicle and blank control, respectively. Data represent mean ± SEM for n=6. *P < 0.01 compared to all other treatments and respective populations. CFU-GM is a methylcellulose based *in vitro* functional assay where HSPC leads to the formation of distinct colonies. Mature cells are unable to form such colonies.

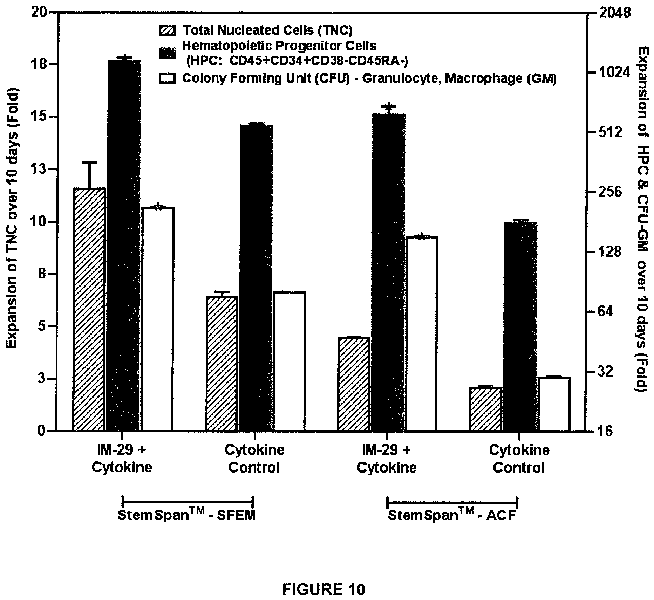

**Figure 10** shows *ex vivo* expansion of total nucleated cells (TNC), hematopoietic progenitor cells (HPC: CD45+CD34+CD38-CD45RA-) and colony forming unit (CFU) - granulocyte, macrophage (GM) when UCB-MNC were cultured in serum-free expansion media (SFEM) or animal-component-free (ACF) media containing 5.0 µM IM-29 in presence of basal cytokines. The expansion cultures lasted for 10 days with SFEM/ACF media, cytokine and IM-29 replenishment being done on day 7. Cytokines alone in SFEM/ACF media served as the blank control. Data represents mean ± SEM for n = 3. **P* < 0.05 compared to Cytokine Control in respective media. The expansion effect of IM-29 was independent of the basal culture media. The use of either a SFEM or ACF media resulted in significantly better expansion of TNC, HPC (CD45+CD34+CD38-CD45RA-), CFU-GM in presence of 5.0 µM of IM-29 compared to the respective cytokine control. SFEM contains bovine serum albumin while ACF is chemically defined.

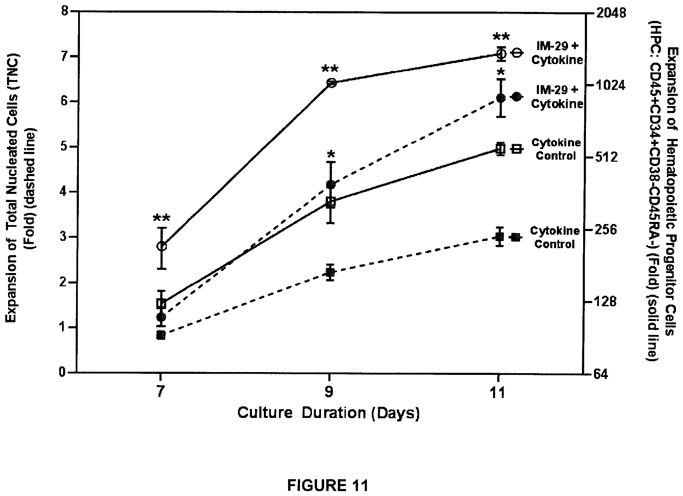

**Figure 11** shows *ex vivo* expansion of UCB by 5.0 µM of IM-29 as a function of the culture duration/period. Fold expansion of hematopoietic progenitor cells (HPC: CD45+CD34+CD38-CD45RA-) (solid line) and total nucleated cells (TNC) (dashed line) in cultures that lasted for 7, 9 and 11 days. Animal component free (ACF) media, cytokine and IM-29 were replenished at day 7 for cultures lasting till day 9 and 11. These set of experiments were carried out with two different UCB samples. Cytokines alone in ACF media served as the blank control. **P* < 0.01 or ***P* < 0.01 compared cytokine control for respective parameter at the mentioned time-point. Data represents mean ± SEM for n = 6. Expansion effect of IM-29 was dependent on the duration of the culture with optimal expansion period being 10 to 11 days.

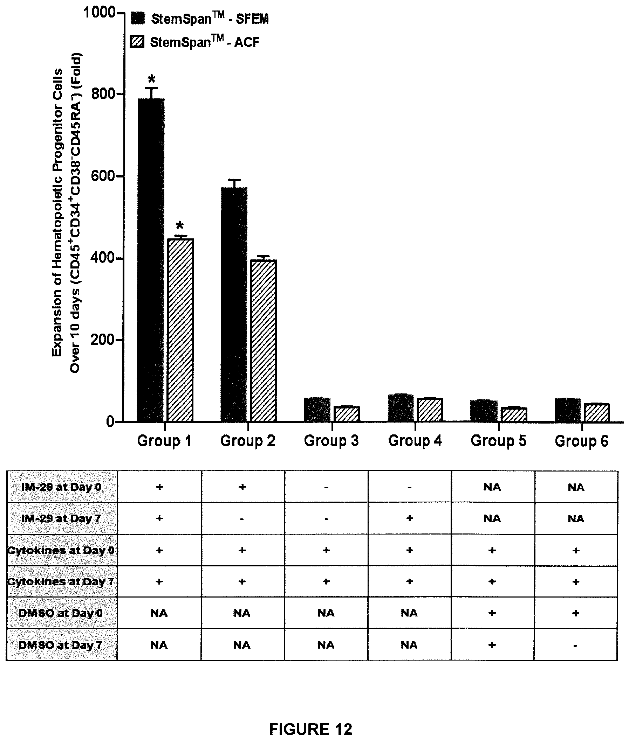

**Figure 12** shows the UCB *ex vivo* expansion effect of IM-29 at 5.0 µM as a function of the time at which it was added to the culture. Fold expansion of HPC in cultures that lasted for 10 days. Serum free expansion media (SFEM) or animal component free (ACF) media, cytokine and IM-29 were added on day 0 and replenished on day 7 as detailed in the table. Data represents mean ± SEM for n= 3. **P* < 0.05 compared to all other groups in respective media. Optimal expansion of UCB HPC was only achieved when IM-29 was supplemented at point of initiating expansion cultures. Expansion was further significantly improved if IM-29 was replenished at day 7 along with media and cytokine.

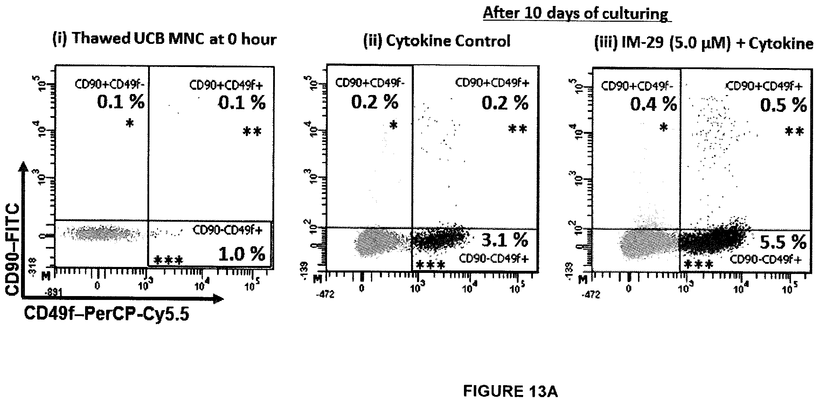

**Figure 13A** shows representative dot plots from flow cytometric analysis depicting (a) CD90+ (region depicted with *); (b) CD90+CD49f+ (region depicted with **) and (c) CD90-CD49f+ (region depicted with ***) population which are subsets of CD45+CD34+ CD38-CD45RA- cells of (i) thawed UCB MNC at 0 hours followed by culturing for 10 days in (ii) cytokine control and (iii) 5.0 µM of IM-29 supplemented with cytokines using serum-free expansion media (SFEM).

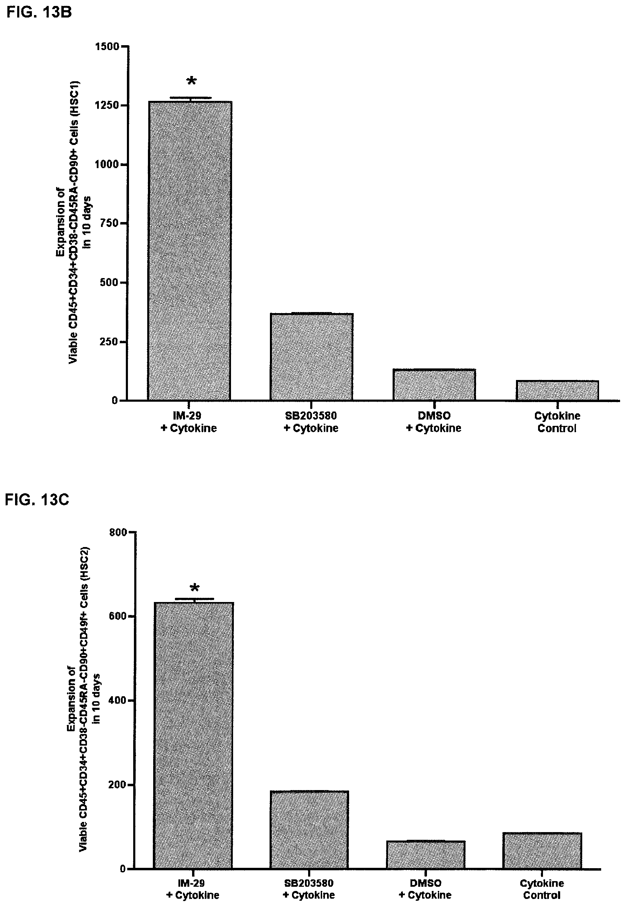

**Figure 13B** shows *ex vivo* expansion of HSC1 with phenotypic expression of CD45+CD34+CD38-CD45RA-CD90+ which is known to engraft immunodeficient mice. The expansion cultures lasted for 10 days, with SFEM, cytokine and IM-29 replenishment being done on day 7. SB203580, DMSO and cytokines alone in SFEM served as the reference compound, vehicle and blank control, respectively. Data represents mean ± SD for n = 3. **P* < 0.001 compared to SB203580, DMSO and Cytokine Control.
**Figure 13C** shows *ex vivo* expansion of HSC2 with phenotypic expression of CD45+CD34+CD38-CD45RA-CD90+CD49f+. The expansion cultures lasted for 10 days, with SFEM, cytokine and IM-29 replenishment being done on day 7. SB203580, DMSO and cytokines alone in SFEM served as the reference compound, vehicle and blank control, respectively. Data represents mean ± SD for n = 3. **P* < 0.001 compared to SB203580, DMSO and Cytokine Control. Detailed investigation of the phenotypic expression of expanded UCB without prior stem cell selection, but using IM-29 showed a significant increase in rare subsets of HSPC defined by antigen expression of (a) CD45+CD34+CD38-CD45RA-CD90+ (HSC1) and (b) CD45+CD34+CD38-CD45RA-CD90+CD49f+ (HSC2). Such subsets from non-manipulated UCB have been reported to possess high self-renewal/repopulating capacity as assessed by *in vivo* serial transplantation studies.

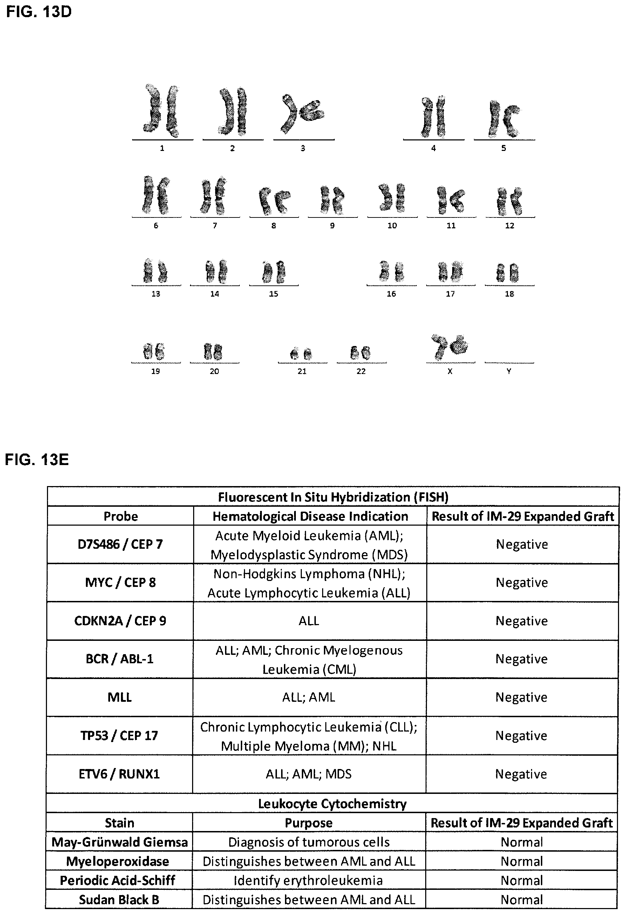

**Figure 13D** shows a representative karyogram of cells expanded from frozen-thawed UCB-MNC in the presence of 5.0 µM of IM-29 in animal component free media (ACF) with basal cytokines. The expansion cultures lasted for 11 days, with ACF, cytokine and IM-29 replenishment being done on day 7. The karyotype of expanded cells is normal compared to non-cultured UCB-MNC.
**Figure 13E** shows results of fluorescence in situ hybridization (FISH) and leukocyte cytochemistry clinical tests conducted on cells expanded from frozen-thawed UCB-MNC in presence of 5.0 µM of IM-29 in animal component free media (ACF) with basal cytokines. The expansion cultures lasted for 11 days, with ACF, cytokine and IM-29 replenishment being done on day 7. The FISH probes used are D7S486 / CEP 7 (for acute myeloid leukemia, AML; myelodysplastic syndrome, MDS); MYC / CEP 8 (for Non-Hodgkins Lymphoma, NHL; acute lymphocytic leukemia, ALL); CDKN2A / CEP 9 (for ALL); BCR / ABL-1 (for ALL; AML; Chronic Myelogenous Leukemia, CML); MLL (for ALL; AML); TP53 / CEP 17 (for chronic lymphocytic leukemia, CLL; multiple myeloma (MM); NHL); and ETV6 / RUNX1 (for AML; ALL; MDS). Leukocyte cytochemistry tests were conducted using the following stains on the cultured cell smears: May-Grünwald Giemsa (detects tumor cells); myeloperoxidase (distinguishes between AML and ALL); periodic acid-schiff (identifies erythroleukemia); and sudan black b (distinguishes between AML and ALL). FISH and leukocyte cytochemistry diagnostic tests suggest that IM-29 expanded cells have no leukemic transformation.

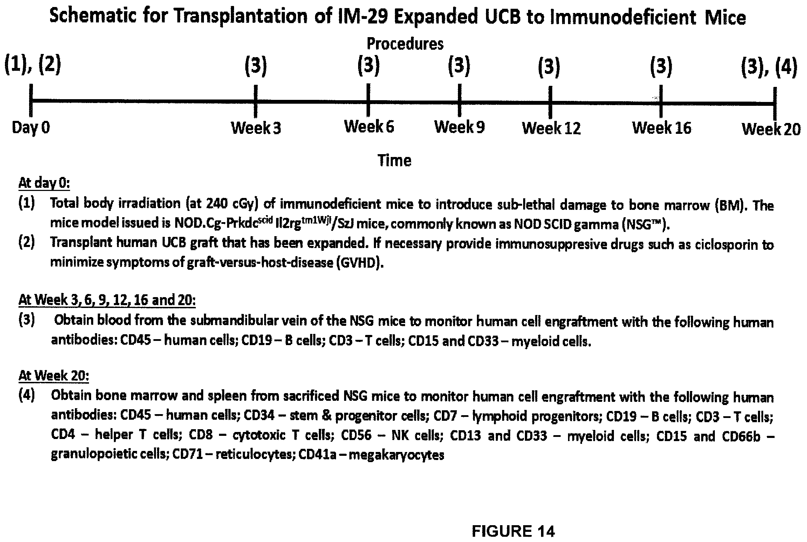

**Figure 14** is a schematic describing the major experimental procedures for transplantation of the IM-29 expanded UCB to immunodeficient mice to evaluate *in vivo* functionality.

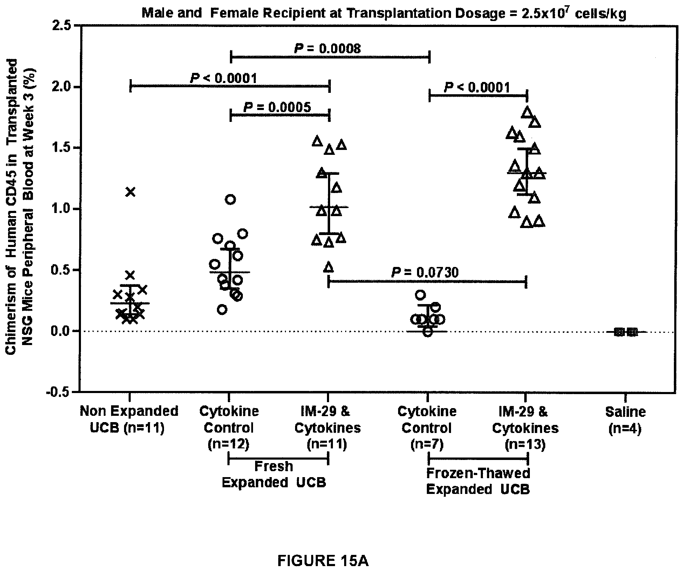

**Figure 15A** shows human CD45 chimerism in peripheral blood (PB) of NOD/SCID/Gamma (NSG) mice at week 3 post-transplantation with non-expanded or expanded UCB. Expansion of the UCB grafts was carried out using the mononuclear fraction (i.e. without CD34 selection) in either the serum-free expansion medium (SFEM) or the animal-free-component (ACF) media that were supplemented with cytokines. Transplantation is carried out as *per* the schematic shown in Figure 14. The absolute cell dose of non-expanded graft was 2.5×10⁷ cells/kg while the expanded grafts (either fresh or frozen-thawed) were transplanted at equivalent cell dosage of 2.5×10⁷ cells/kg. The scatter plot represents the human CD45 chimerism of individual animals and depicts the geometric mean with 95% confidence interval (CI) of respective treatments. *P* values generated from Student's t-test amongst indicated experimental groups are shown in the graph for the stated n values.

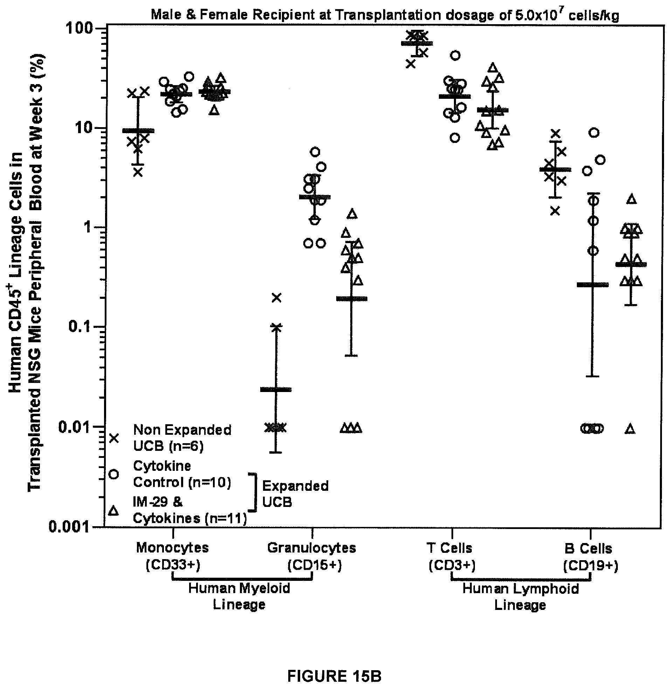

**Figure 15B** shows the lineage commitment of the human CD45 cells that are present in the peripheral blood (PB) of NSG mice at week 3 post-transplantation as *per* Figure 15A. The absolute cell dose of non-expanded graft was 5.0×10⁷ cells/kg while the expanded grafts were transplanted at equivalent cell dosage of 5.0×10⁷ cells/kg. The scatter plot represents the proportion of monocytes (CD45+CD33+), granulocytes (CD45+CD15+), T cells (CD45+CD3+) and B cells (CD45+CD19+) present amongst the total human cells in each individual animals and depicts the geometric mean with 95% confidence interval (CI) of respective treatments.

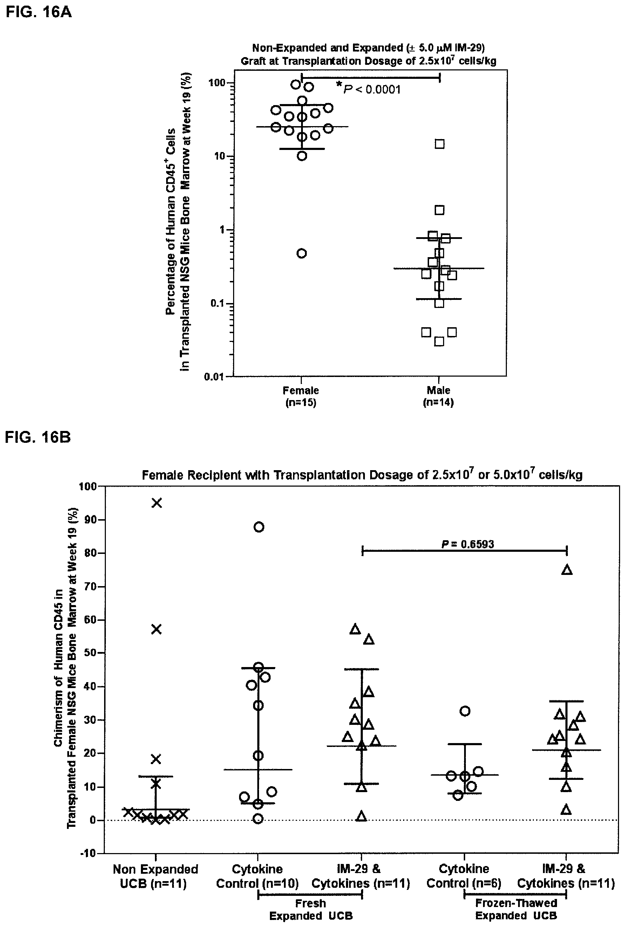

**Figure 16A** shows the results of UCB mononucleated cells expanded under different culture conditions transplanted into sub-lethally irradiated immunodeficient NOD/SCID/Gamma (NSG) mice, whereby the percentage of human CD45+ cells and lineage commitment of the engrafted human cells in the bone marrow of the NSG mice was determined after 19 weeks post-transplantation. In this data set, the chimerism from mice receiving non-expanded or expanded graft (± 5.0 µM IM-29) is segregated by gender of the recipient mice and not by graft type. The absolute cell dose of non-expanded graft was 2.5×10⁷ cells/kg while the expanded grafts were transplanted at equivalent cell dosage of 2.5×10⁷ cells/kg. The scatter plot represents the human CD45 chimerism of individual animals and depicts the geometric mean with 95% confidence interval (CI) of respective gender.
**Figure 16B** shows human CD45 chimerism in bone marrow (BM) of female NSG mice at week 19 post-transplantation per Fig. 16A. The absolute cell dose of non-expanded graft was either 2.5×10⁷ cells/kg or 5.0×10⁷ cells/kg while the expanded grafts (either fresh or frozen-thawed) were transplanted at equivalent cell dosage of 2.5×10⁷ cells/kg or 5.0×10⁷ cells/kg. The scatter plot represents the human CD45 chimerism of individual animals and depicts the geometric mean with 95% confidence interval (CI) of respective treatments. *P* values generated from Student's t-test amongst indicated experimental groups are shown in the graph for the stated n values.

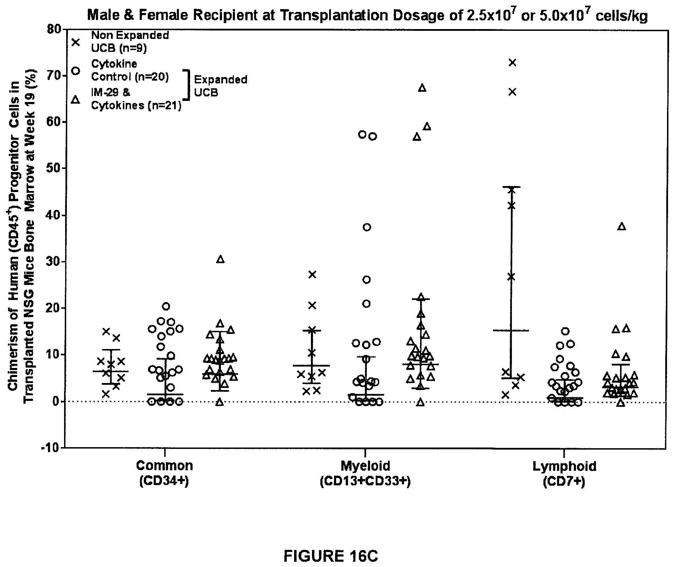

**Figure 16C** shows the proportion of progenitor cells present amongst the total human cells in bone marrow (BM) of male and female NSG mice at week 19 post-transplantation per Fig. 16A. The absolute cell dose of non-expanded graft was either 2.5×10⁷ cells/kg or 5.0×10⁷ cells/kg while the expanded grafts were transplanted at equivalent cell dosage of 2.5×10⁷ cells/kg or 5.0×10⁷ cells/kg. The scatter plot represents the common progenitors (CD45+CD34+), myeloid (CD13+CD33+) and lymphoid (CD45+CD7+) progenitors of individual animals and depicts the geometric mean with 95% confidence interval (CI) of respective treatments.

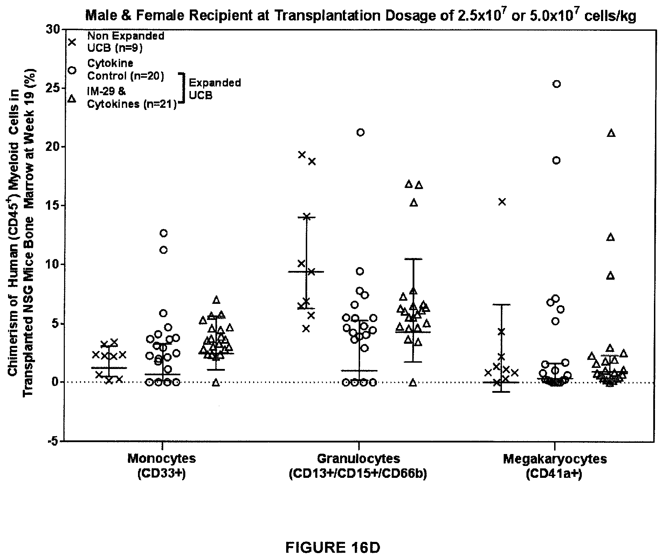

**Figure 16D** shows the proportion of myeloid cells present amongst the total human cells in bone marrow (BM) of male and female NSG mice at week 19 post-transplantation as *per* Fig. 16A. The absolute cell dose of non-expanded graft was either 2.5×10⁷ cells/kg or 5.0×10⁷ cells/kg while the expanded grafts were transplanted at equivalent cell dosage of 2.5×10⁷ cells/kg or 5.0×10⁷ cells/kg. The scatter plot represents the monocytes (CD45+CD33+), granulocytes (CD45+CD13+/CD15+/CD66b+) and megakaryocytes (CD45+CD41a+) of individual animals and depicts the geometric mean with 95% confidence interval (CI) of respective treatments.

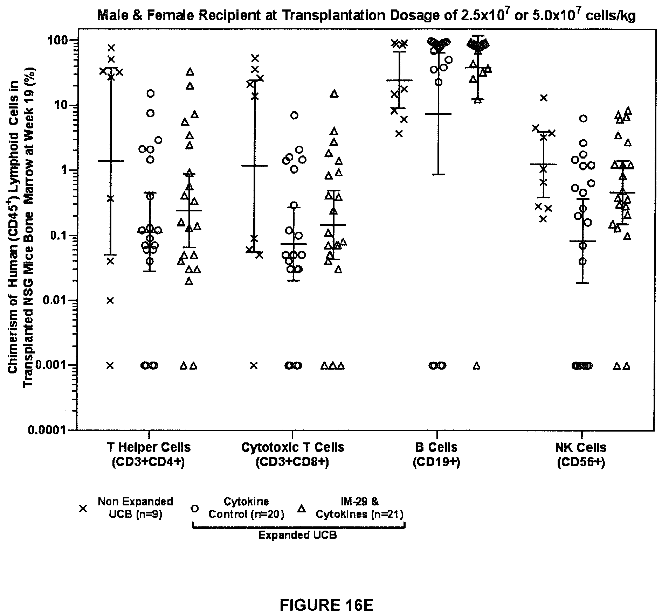

**Figure 16E** shows the proportion of lymphoid cells present amongst the total human cells in bone marrow (BM) of male and female NSG mice at week 19 post-transplantation as *per* Fig. 16A. The absolute cell dose of non-expanded graft was either 2.5×10⁷ cells/kg or 5.0×10⁷ cells/kg while the expanded grafts were transplanted at equivalent cell dosage of 2.5×10⁷ cells/kg or 5.0×10⁷ cells/kg. The scatter plot represents the T helper cells (CD45+CD3+CD4+), cytotoxic T cells (CD45+CD3+CD8+), B cells (CD45+CD19+) and NK cells (CD45+CD56+) progenitors of individual animals and depicts the geometric mean with 95% confidence interval (CI) of respective treatments. Among the various groups, UCB expanded with IM-29 (5.0 µM) and cytokines irrespective of basal culture media seems to give the best human CD45 chimerism in the mouse recipients thus far, suggesting both faster engraftment and ability to maintain long term hematopoiesis.

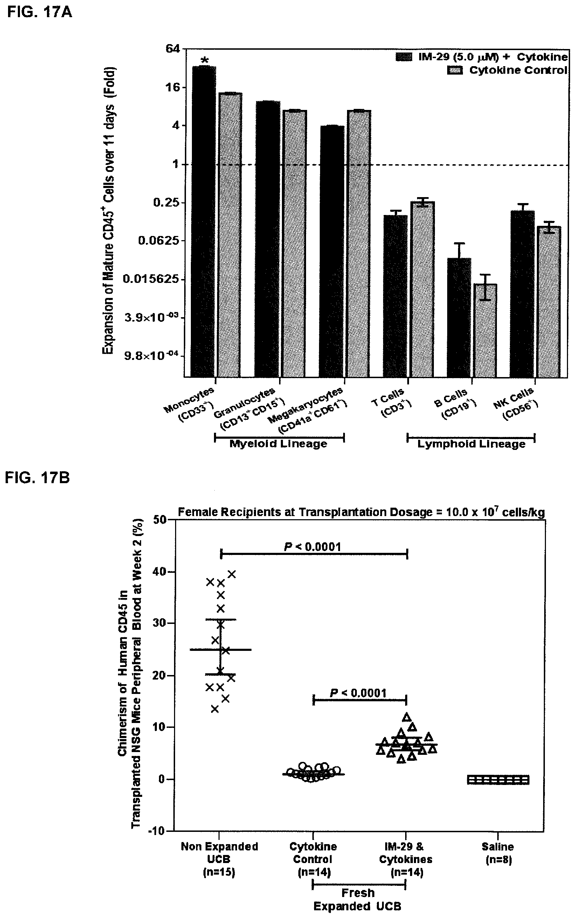

**Figures 17A-17H** show that UCB mononucleated cells (MNC) expanded in the presence of 5.0 µM of IM-29 and basal cytokines primarily generated myeloid progenitors and mature cells which, when transplanted into sub-lethally irradiated immunodeficient NOD/SCID/Gamma (NSG) mice, resulted in early engraftment of myeloid and progenitor cells in peripheral blood (PB) and bone marrow (BM) while confirming long-term human multi-lineage reconstitution of the NSG BM.
**Figure 17A** shows the expansion of mature myeloid and lymphoid lineage cells in IM-29 and cytokine control cultures over 11 days. These MNC expansion cultures were supplemented with ACF media, cytokine and IM-29 at day 7. Myeloid lineage consisted of CD45+CD33+ monocytes, CD45+CD13+CD15+ granulocytes and CD45+CD41a+CD61+ megakaryocytes. Lymphoid lineage consisted of CD45+CD3+ T cells, CD45+CD19+ B cells and CD45⁺CD56⁺ NK cells. **P* < 0.001 compared to respective population in each treatment group. Data represents mean ± SD for n = 3.
**Figure 17B** shows a scatter plot of human CD45 chimerism in peripheral blood (PB) of NSG mice at week 2 post-transplantation. The absolute cell dose of non-expanded graft was 10.0×10⁷ cells/kg while the expanded grafts were transplanted at equivalent cell dosage of 10.0×10⁷ cells/kg. The scatter plot represents the human CD45 chimerism of individual animals and depicts the geometric mean with 95% confidence interval (CI) of respective treatments. *P* values generated from Student's t-test amongst indicated experimental groups are shown in the graph for the stated n values.

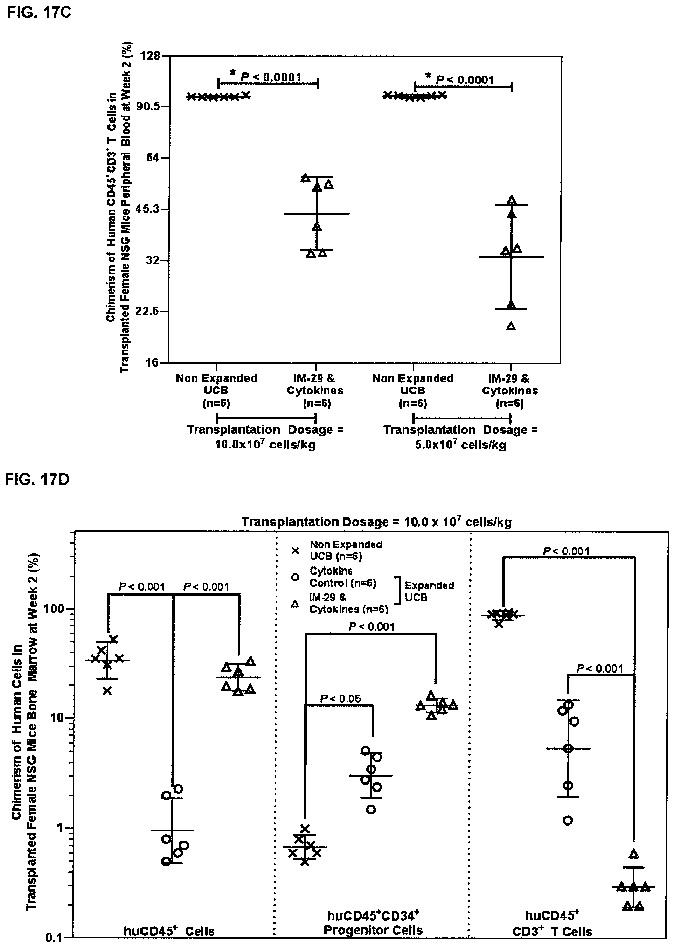

**Figure 17C** shows a scatter plot of human CD45+CD3+ T cell chimerism in peripheral blood (PB) of NSG mice at week 2 post-transplantation. The absolute cell dose of non-expanded graft was 10.0×10⁷ or 5.0×10⁷ cells/kg while the IM-29 expanded grafts were transplanted at equivalent cell dosage of 10.0×10⁷ or 5.0×10⁷ cells/kg. The scatter plot represents the human T cell chimerism of individual animals and depicts the geometric mean with 95% confidence interval (CI) of respective treatments. *P* values generated from Student's t-test amongst indicated experimental groups are shown in the graph for the stated n values.
**Figure 17D** shows a scatter plot of human CD45+, CD45+CD34+ progenitor and CD45+CD3+ T cell chimerism in bone marrow (BM) of female NSG mice at week 2 post-transplantation. The absolute cell dose of non-expanded graft was 10.0×10⁷ cells/kg while the expanded grafts were transplanted at equivalent cell dosage of 10.0×10⁷ cells/kg. The scatter plot represents the human CD45+, CD45+CD34+ progenitor and CD45+CD3+ T cell chimerism of individual animals and depicts the geometric mean with 95% confidence interval (CI) of respective treatments. *P* values generated from Student's t-test amongst indicated experimental groups are shown in the graph for the stated n values.

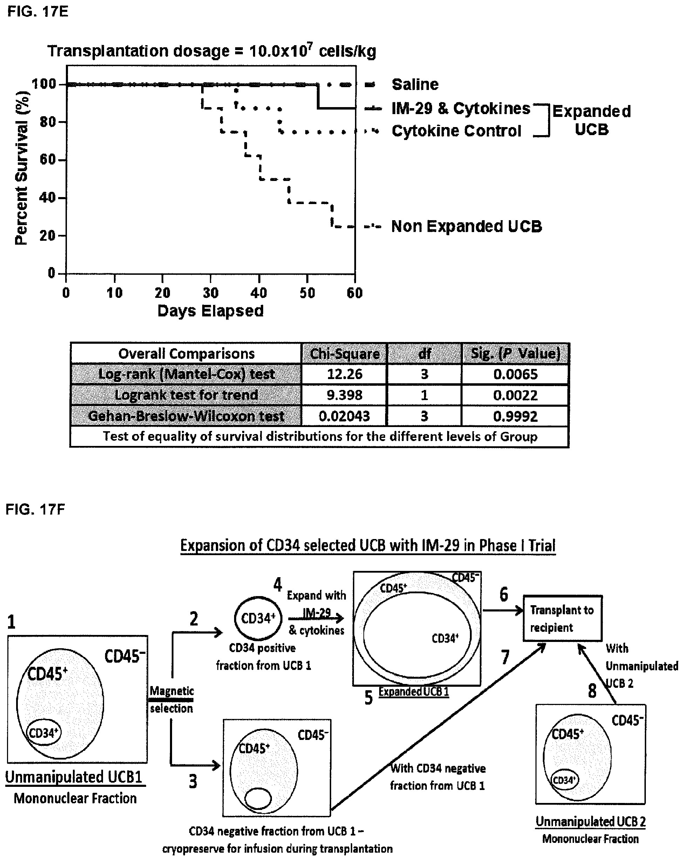

**Figure 17E** shows a Kaplan-Meier survival curve of the NSG mice transplanted with IM-29 or cytokine expanded UCB-MNC and non-expanded graft over 60-days observation period. The absolute cell dose of non-expanded graft was 10.0×10⁷ cells/kg while the expanded grafts were transplanted at equivalent cell dosage of 10.0×10⁷ cells/kg. The overall statistical comparison for the experimental groups is also shown.
**Figure 17F** shows a schematic describing the transplantation regimen of IM-29 expanded UCB when expansion cultures are initiated with magnetically purified CD34+ cells. To enable IM-29 expansion protocol to translate into a phase I clinical trial, it is necessary to prove the efficacy of the protocol to expand purified HSPC.

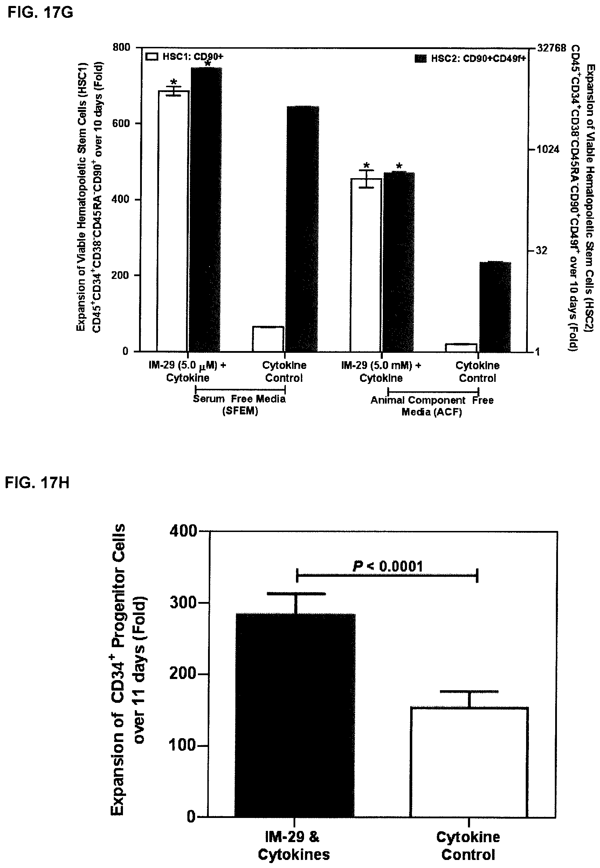

**Figure 17G** shows the level of *ex vivo* expansion of HSPC with phenotype of CD45+CD34+CD38-CD45RA-CD90+CD49f- (HSC1) or CD45+CD34+CD38-CD45RA-CD90+CD49f+ (HSC2) when purified immature HSPC with phenotype CD45+CD34+CD38-were cultured in serum-free expansion media (SFEM) or animal component free (ACF) media containing 5.0 µM IM-29 in presence of basal cytokines. The expansion cultures lasted for 10 days with SFEM/ACF media, cytokine and IM-29 replenishment being done on day 7. Cytokines alone in SFEM/ACF media served as the blank control. **P* < 0.05 compared to Cytokine Control in respective media. Data represents mean ± SD for n = 3.
**Figure 17H** shows the level of *ex vivo* expansion of CD34⁺ cells when cultures were initiated with magnetically purified CD34⁺ cells. The expansion cultures lasted for 11 days with ACF media, cytokine and 5.0 µM IM-29 replenishment being done on day 7. **P* < 0.0001 compared to Cytokine Control. Data represents mean ± SEM for n = 6.

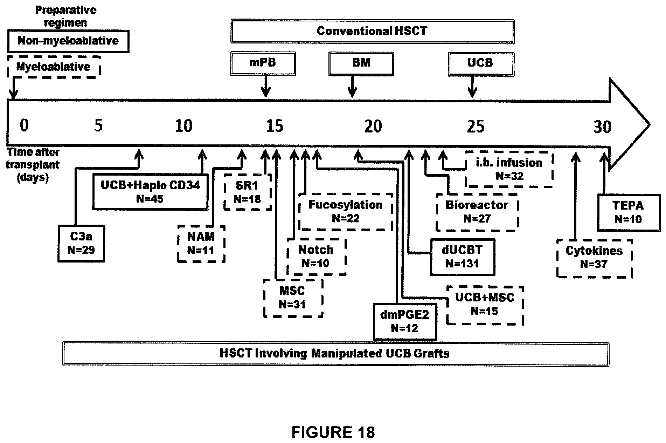

**Figure 18** is a schematic summarizing the median time to neutrophil recovery in completed clinical trials involving manipulated UCB grafts. The median time to neutrophil recovery is the primary indictor of success for hematopoietic stem cell transplantation (HSCT) which can be carried out using matched donor bone marrow (BM), mobilized peripheral blood (mPB) or umbilical cord blood (UCB) as the source of graft. The median time for neutrophil recovery, indicated by individual upward/downward solid arrows towards the post-transplant timeline, which is represented by the central right arrow, for conventional HSCT (shown above the post-transplant timeline) using mPB, BM or UCB is 14, 18 and 25 days, respectively. For HSCT using manipulated UCB grafts (shown below the post-transplant timeline), patients either received non-myeloablative/reduced intensity (solid boxes) or myeloablative (dashed boxes) conditioning. The number of patients enrolled in each of these trials is represented by the N value shown in each box. The trials can be categorized into two broad categories (as shown in Tables 1 and 2):

Increasing absolute number of infused total nucleated cells:
(a) Dual unit UCBT (dUCBT);
(b) single unit UCBT combined with haplo-identical CD34+ cells (UCB+Haplo CD34+);
(c) *Ex vivo* expansion of a single unit of UCB which was co-transplanted with an unmanipulated unit. To date, clinical expansion has been done using:
   (i) Cytokine;
   (ii) Bioreactors;
   (iii) Co-culture with mesenchymal stromal cells (MSC);
   (iv) Biomolecules such as Notch;
   (v) Nicotinamide (NAM - SIRT1 inhibitor);
   (vi) Stemregenin 1 (SR1 - antagonist of aryl hydrocarbon receptor);
   (vii) Tetraethylenepentamine (TEPA - copper chelator).

Improving homing of infused/transplanted cells:
(a) Intrabone marrow infusion (i.b. infusion) of singe UCB unit with or without intravenous infusion of another unmanipulated unit;
(b) Intravenous (i.v.) co-administration of single UCB along with MSC (UCB+MSC);
(c) Priming of an UCB unit with various chemicals and bio-molecules such as:
   (i) dimethyl-prostaglandin E2 (dmPGE2);
   (ii) complement fragment 3a (C3a); and
   (iii) fucosylation in the setting of dual unit UCBT.

### DETAILED DESCRIPTION OF THE INVENTION

Bibliographic references mentioned in the present specification are for convenience listed in the form of a list of references and added at the end of the examples. The whole content of such bibliographic references is herein incorporated by reference.

### Definitions

For convenience, certain terms employed in the specification, examples and appended claims are collected here.

The term "comprising" is herein defined to be that where the various components, ingredients, or steps, can be conjointly employed in practising the present invention. Accordingly, the term "comprising" encompasses the more restrictive terms "consisting essentially of" and "consisting of."

The term "halo", when used herein, includes references to fluoro, chloro, bromo and iodo.

Unless otherwise stated, the term "aryl" when used herein includes C₆₋₁₆ (such as C₆₋₁₄ or C₆₋₁₀) aryl groups. Such groups may be monocyclic, bicyclic or tricyclic and have between 6 and 16 (e.g. between 6 and 14, or between 6 and 10) ring carbon atoms, in which at least one ring is aromatic. The point of attachment of aryl groups may be *via* any atom of the ring system. However, when aryl groups are bicyclic or tricyclic, they are linked to the rest of the molecule *via* an aromatic ring. C₆₋₁₆ aryl groups include phenyl, naphthyl, phenanthracenyl and pyrenyl and the like, such as 1,2,3,4-tetrahydronaphthyl, indanyl, indenyl and fluorenyl. Embodiments of the invention that may be mentioned include those in which aryl is phenyl, naphthyl, phenanthracenyl or pyrenyl.

Unless otherwise stated, the term "hereroaromatic" when used herein includes 6- to 10-membered heteroaromatic ring systems that may be monocyclic, bicyclic or tricyclic and have from one to six (e.g. one to three, such as one) heteroatoms selected from O, N and S. The heteroaromatic ring system contains at least one ring that is aromatic in character and when the ring system is bicyclic or tricyclic, the ring system is attached to the rest of the molecule *via* a heteroaromatic ring.

Monocyclic heteroaromatic groups include, for example, pyridinyl, pyrazinyl, pyrimidinyl, pyridazinyl, triazinyl and the like. Bicyclic heteroaromatic groups include, for example, benzimidazolyl, benzisothiazolyl, benzisoxazolyl, benzofuranyl, benzoxazolyl, benzopyrazolyl, benzothiazolyl, benzothienyl, indazolyl, indolyl, isoindolyl, purinyl, pyrrolo[2,3-6]pyridinyl, pyrrolo[5,1-6]pyridinyl, pyrrolo[2,3-c]pyridinyl, 4,5,6,7-tetrahydrobenzimidazolyl, 4,5,6,7-tetrahydrobenzopyrazolyl, thieno[5,1-c]pyridinyl and the like, which bicyclic heteroaromatic groups are attached to the rest of the molecule via an atom in the 5- membered ring. Tricyclic heteroaromatic groups include acridinyl, phenazinyl

Heterocyclic groups may be fully saturated, partly unsaturated, wholly aromatic or partly aromatic in character. Values of heterocyclic groups that may be mentioned include 1-azabicyclo[2.2.2]octanyl, benzimidazolyl, benzisothiazolyl, benzisoxazolyl, benzodioxanyl, benzodioxepanyl, benzodioxepinyl, benzodioxolyl, benzofuranyl, benzofurazanyl, benzo[c]isoxazolidinyl, benzomorpholinyl, 2,1,3-benzoxadiazolyl, benzoxazinyl (including 3,4-dihydro-2*H*-1,4-benzoxazinyl), benzoxazolidinyl, benzoxazolyl, benzopyrazolyl, benzo[*e*]pyrimidine, 2,1,3-benzothiadiazolyl, benzothiazolyl, benzothienyl, benzotriazolyl, carbazolyl, chromanyl, chromenyl, cinnolinyl, 2,3-dihydrobenzimidazolyl, 2,3-dihydrobenzo[6]furanyl, 1,3-dihydrobenzo[c]furanyl, 1,3-dihydro-2,1-benzisoxazolyl, 2,3-dihydropyrrolo[2,3-*b*]pyridinyl, dioxanyl, hexahydropyrimidinyl, imidazo[1,2-*a*]pyridinyl, imidazo[2,3-*b*]thiazolyl, indazolyl, indolinyl, indolyl, isobenzofuranyl, isochromanyl, isoindolinyl, isoindolyl, isoquinolinyl, isothiaziolyl, isothiochromanyl, isoxazolidinyl, isoxazolyl, morpholinyl, naphtho[1,2-*b*]furanyl, naphthyridinyl (including 1,6-naphthyridinyl or, particularly, 1,5-naphthyridinyl and 1,8-naphthyridinyl), 1,2- or 1,3-oxazinanyl, phenazinyl, phenothiazinyl, phthalazinyl, piperazinyl, piperidinyl, pteridinyl, purinyl, pyranyl, pyrazinyl, pyridazinyl, pyridinyl, pyrimidinyl, pyrrolo[2,3-*b*]pyridinyl, pyrrolo[5,1-*b*]pyridinyl, pyrrolo[2,3-*c*]pyridinyl, quinazolinyl, quinolinyl, quinolizinyl, quinoxalinyl, 4,5,6,7-tetrahydrobenzimidazolyl, 4,5,6,7-tetrahydrobenzopyrazolyl, 5,6,7,8-tetrahydrobenzo[e]pyrimidine, tetrahydroisoquinolinyl (including 1,2,3,4-tetrahydroisoquinolinyl and 5,6,7,8-tetrahydroisoquinolinyl), tetrahydropyranyl, 3,4,5,6-tetrahydropyridinyl, 1,2,3,4-tetrahydropyrimidinyl, 3,4,5,6-tetrahydropyrimidinyl, tetrahydroquinolinyl (including 1,2,3,4-tetrahydroquinolinyl and 5,6,7,8-tetrahydroquinolinyl), thieno[5,1-*c*]pyridinyl, thiochromanyl, 1,3,4-triazolo[2,3-*b*]pyrimidinyl, xanthenyl and the like.

References herein (in any aspect or embodiment of the invention) to compounds of formula I include references to such compounds *per se,* to tautomers of such compounds, as well as to salts or solvates of such compounds.

Salts that may be mentioned include acid addition salts and base addition salts. Such salts may be formed by conventional means, for example by reaction of a free acid or a free base form of a compound of formula I with one or more equivalents of an appropriate acid or base, optionally in a solvent, or in a medium in which the salt is insoluble, followed by removal of said solvent, or said medium, using standard techniques (e.g. *in vacuo,* by freeze-drying or by filtration). Salts may also be prepared by exchanging a counter-ion of a compound of formula I in the form of a salt with another counter-ion, for example using a suitable ion exchange resin.

Examples of salts include acid addition salts derived from mineral acids and organic acids, and salts derived from metals such as sodium, magnesium, or preferably, potassium and calcium.

Examples of acid addition salts include acid addition salts formed with acetic, 2,2-dichloroacetic, adipic, alginic, aryl sulphonic acids (e.g. benzenesulphonic, naphthalene-2-sulphonic, naphthalene-1,5-disulphonic and p-toluenesulphonic), ascorbic (e.g. L-ascorbic), L-aspartic, benzoic, 4-acetamidobenzoic, butanoic, (+) camphoric, camphor-sulphonic, (+)-(1S)-camphor-10-sulphonic, capric, caproic, caprylic, cinnamic, citric, cyclamic, dodecylsulphuric, ethane-1,2-disulphonic, ethanesulphonic, 2-hydroxyethanesulphonic, formic, fumaric, galactaric, gentisic, glucoheptonic, gluconic (e.g. D-gluconic), glucuronic (e.g. D-glucuronic), glutamic (e.g. L-glutamic), α-oxoglutaric, glycolic, hippuric, hydrobromic, hydrochloric, hydriodic, isethionic, lactic (e.g. (+)-L-lactic and (±)-DL-lactic), lactobionic, maleic, malic (e.g. (-)-L-malic), malonic, (±)-DL-mandelic, metaphosphoric, methanesulphonic, 1-hydroxy-2-naphthoic, nicotinic, nitric, oleic, orotic, oxalic, palmitic, pamoic, phosphoric, propionic, L-pyroglutamic, salicylic, 4-amino-salicylic, sebacic, stearic, succinic, sulphuric, tannic, tartaric (e.g.(+)-L-tartaric), thiocyanic, undecylenic and valeric acids.

Particular examples of salts are salts derived from mineral acids such as hydrochloric, hydrobromic, phosphoric, metaphosphoric, nitric and sulphuric acids; from organic acids, such as tartaric, acetic, citric, malic, lactic, fumaric, benzoic, glycolic, gluconic, succinic, arylsulphonic acids; and from metals such as sodium, magnesium, or preferably, potassium and calcium.

As mentioned above, also encompassed by formula I are any solvates of the compounds and their salts. Preferred solvates are solvates formed by the incorporation into the solid state structure (e.g. crystal structure) of the compounds of the invention of molecules of a non-toxic pharmaceutically acceptable solvent (referred to be low as the solvating solvent). Examples of such solvents include water, alcohols (such as ethanol, isopropanol and butanol) and dimethylsulphoxide. Solvates can be prepared by recrystallising the compounds of the invention with a solvent or mixture of solvents containing the solvating solvent. Whether or not a solvate has been formed in any given instance can be determined by subjecting crystals of the compound to analysis using well known and standard techniques such as thermogravimetric analysis (TGA), differential scanning calorimetry (DSC) and X-ray crystallography.

The solvates can be stoichiometric or non-stoichiometric solvates. Particularly preferred solvates are hydrates, and examples of hydrates include hemihydrates, monohydrates and dihydrates.

For a more detailed discussion of solvates and the methods used to make and characterise them, see Bryn et al., Solid-State Chemistry of Drugs, Second Edition, published by SSCI, Inc of West Lafayette, IN, USA, 1999, ISBN 0-967-06710-3.

Compounds of formula I, as well as pharmaceutically acceptable salts, solvates and pharmaceutically functional derivatives of such compounds are, for the sake of brevity, hereinafter referred to together as the "compounds of formula I".

Compounds of formula I may contain double bonds and may thus exist as *E* (entgegen) and *Z* (zusammen) geometric isomers about each individual double bond. All such isomers and mixtures thereof are included within the scope of the invention.

Compounds of formula I may exist as regioisomers and may also exhibit tautomerism. All tautomeric forms and mixtures thereof are included within the scope of the invention.

Compounds of formula I may contain one or more asymmetric carbon atoms and may therefore exhibit optical and/or diastereoisomerism. Diastereoisomers may be separated using conventional techniques, e.g. chromatography or fractional crystallisation. The various stereoisomers may be isolated by separation of a racemic or other mixture of the compounds using conventional, e.g. fractional crystallisation or HPLC, techniques. Alternatively the desired optical isomers may be made by reaction of the appropriate optically active starting materials under conditions which will not cause racemisation or epimerisation (i.e. a 'chiral pool' method), by reaction of the appropriate starting material with a 'chiral auxiliary' which can subsequently be removed at a suitable stage, by derivatisation (i.e. a resolution, including a dynamic resolution), for example with a homochiral acid followed by separation of the diastereomeric derivatives by conventional means such as chromatography, or by reaction with an appropriate chiral reagent or chiral catalyst all under conditions known to the skilled person. All stereoisomers and mixtures thereof are included within the scope of the invention.

Further embodiments of the invention that may be mentioned include those in which the compound of formula I is isotopically labelled. However, other, particular embodiments of the invention that may be mentioned include those in which the compound of formula I is not isotopically labelled.

The term "isotopically labelled", when used herein includes references to compounds of formula I in which there is a non-natural isotope (or a non-natural distribution of isotopes) at one or more positions in the compound. References herein to "one or more positions in the compound" will be understood by those skilled in the art to refer to one or more of the atoms of the compound of formula I. Thus, the term "isotopically labelled" includes references to compounds of formula I that are isotopically enriched at one or more positions in the compound.

The isotopic labelling or enrichment of the compound of formula I may be with a radioactive or non-radioactive isotope of any of hydrogen, carbon, nitrogen, oxygen, sulfur, fluorine, chlorine, bromine and/or iodine. Particular isotopes that may be mentioned in this respect include ²H, ³H, ¹¹C, ¹³C, ¹⁴C, ¹³N, ¹⁵N, ¹⁵O, ¹⁷O, ¹⁸O, ³⁵S, ¹⁸F, ³⁷Cl, ⁷⁷Br, ⁸²Br and ¹²⁵I).

When the compound of formula I is labelled or enriched with a radioactive or nonradioactive isotope, compounds of formula I that may be mentioned include those in which at least one atom in the compound displays an isotopic distribution in which a radioactive or non-radioactive isotope of the atom in question is present in levels at least 10% (e.g. from 10% to 5000%, particularly from 50% to 1000% and more particularly from 100% to 500%) above the natural level of that radioactive or non-radioactive isotope.

Other compounds of formula I may be prepared in accordance with techniques that are well known to those skilled in the art, for example as described herein in the examples section.

Substituents, such as R² in final compounds of formula I (or precursors thereto and other relevant intermediates) may be modified one or more times, after or during the processes described hereinafter by way of methods that are well known to those skilled in the art. Examples of such methods include substitutions, reductions (e.g. carbonyl bond reductions in the presence of suitable and, if necessary, chemoselective, reducing agents such as LiBH₄ or NaBH₄), oxidations, alkylations, acylations, hydrolyses, esterifications, and etherifications. The precursor groups can be changed to a different such group, or to the groups defined in formula I, at any time during the reaction sequence.

Compounds of the invention may be isolated from their reaction mixtures using conventional techniques (e.g. recrystallisation, column chromatography, preparative HPLC, etc.).

In the processes described hereinafter, the functional groups of intermediate compounds may need to be protected by protecting groups.

The protection and deprotection of functional groups may take place before or after a reaction in the above-mentioned schemes.

Protecting groups may be removed in accordance with techniques that are well known to those skilled in the art and as described hereinafter. For example, protected compounds/intermediates described hereinafter may be converted chemically to unprotected compounds using standard deprotection techniques.

The type of chemistry involved will dictate the need, and type, of protecting groups as well as the sequence for accomplishing the synthesis.

The use of protecting groups is fully described in *"*Protective Groups in Organic Chemistry", edited by J W F McOmie, Plenum Press (1973), and *"*Protective Groups in Organic Synthesis", 3rd edition, T.W. Greene & P.G.M. Wutz, Wiley-Interscience (1999).

As used herein, the term "functional groups" means, in the case of unprotected functional groups, hydroxy-, thiolo-, amino function, carboxylic acid and, in the case of protected functional groups, lower alkoxy, N-, O-, S- acetyl, carboxylic acid ester.

The term "treatment", as used in the context of the invention refers to prophylactic, ameliorating, therapeutic or curative treatment.

The term "subject" is herein defined as vertebrate, particularly mammal, more particularly human. For purposes of research, the subject may particularly be at least one animal model, e.g., a mouse, rat and the like. For example, for treatment of malignant and benign blood disorders the subject may be a human with acute myeloid leukemia.

A person skilled in the art will appreciate that the present invention may be practised without undue experimentation according to the method given herein. The methods, techniques and chemicals are as described in the references given or from protocols in standard biotechnology and molecular biology text books.

According to a preferred aspect, the present invention provides a method for *ex vivo* expansion of a total nucleated cells and/or a subset of a CD45+CD34+ hematopoietic stem cells and progenitor cells component of an umbilical cord blood, bone marrow or mobilized peripheral blood sample comprising the steps of:
(i) culturing a total nucleated cells or a mononucleated cell fraction or CD45+CD34+ hematopoietic stem cells and progenitor cells component of the sample in media; and
(ii) contacting the cell(s) of step (i) with a composition comprising at least one azole-based small molecule,
   wherein the at least one azole-based small molecule is represented by formula (I), 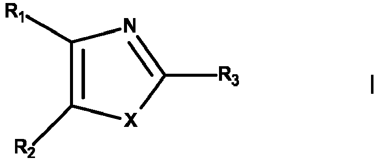 wherein:
   X represents NR₄ or O, where R₄ is H or methyl;
   R₁ represents phenyl or pyridinyl (which are unsubstituted or substituted with one or more substituents selected from Cl, Br, F and methyl (which latter group is unsubstituted or substituted with one or more groups selected from F)).

R₂ represents phenyl, pyridyl or dihydropyranyl (which are unsubstituted or substituted with one or more substituents selected from Br, Cl, F or methyl (which latter group is unsubstituted or substituted with one or more groups selected from F).

R₃ represents H or naphthyl which group is unsubstituted or substituted with one or more groups selected from CI, F, and OR₅ where R₅ is H or methyl, or
salts and solvates thereof.

Although there are benefits in using non-enriched samples, the expansion method may also use an enriched/pre-selected CD34+ cell fraction from umbilical cord blood, bone marrow or peripheral blood samples when used to initiate cultures in the presence of at least one azole-based small molecule.

In another preferred embodiment of the invention, the at least one azole-based small molecule is selected from the group:
(i) 4-[2-(1-fluoronaphthalen-2-yl)-4(5)-(4-fluorophenyl)-1*H*-imidazol-5(4)-yl]pyridine;
(ii) 4-[2-(1-fluoronaphthalen-2-yl)-4-(*m*-tolyl)-1*H*-imidazol-5-yl]pyridine;
(iii) 4-[2-(naphthalen-2-yl)-4(5)-(*m*-tolyl)-1H-imidazol-5(4)-yl]pyridine;
(iv) 4-[2-(naphthalen-2-yl)-4(5)-(4-fluorophenyl)-1*H*-imidazol-5(4)-yl]pyridine;
(v) 4-[2-(1-bromonaphthalen-2-yl)-4(5)-(4-fluorophenyl)-1*H*-imidazol-5(4)-yl]pyridine;
(vi) 4-[2-(1-fluoronaphthalen-2-yl)-4-[3-(trifluoromethyl)phenyl]-1*H*-imidazol-5-yl]pyridine;
(vii) 2-(1-fluoronaphthalen-2-yl)-4-(pyridin-4-yl)-5-(*m*-tolyl)oxazole;
(viii) 5(4)-(3,6-dihydro-2*H*-pyran-4-yl)-2-(1-fluoronaphthalen-2-yl)-4(5)-(*m*-tolyl)-1*H-*imidazole;
(ix) 5(4)-(3,6-dihydro-2*H*-pyran-4-yl)-2-(6-methoxynaphthalen-2-yl)-4(5)-(*m*-tolyl)-1*H*-imidazole; and
(x) 5(4)-(3,6-dihydro-2*H*-pyran-4-yl)-2-(1-fluoronaphthalen-2-yl)-4(5)-(4-fluorophenyl)-1*H*-imidazole;
(xi) 4-(4(5)-(4-fluorophenyl)-2-(7-methoxynaphthalen-2-yl)-1*H*-imidazol-5(4)-yl)pyridine;
(xii) 4-[4(5)-(*m*-tolyl)-1*H*-imidazol-5(4)-yl]pyridine; and
(xiii) 4-[4(5)-(4-fluorophenyl)-1H-imidazol-5(4)-yl]pyridine.

In another preferred embodiment of the invention, the at least one azole-based small molecule is selected from the group:
(i) 4-[2-(1-fluoronaphthalen-2-yl)-4(5)-(4-fluorophenyl)-1*H*-imidazol-5(4)-yl]pyridine;
(ii) 4-[2-(1-fluoronaphthalen-2-yl)-4-(*m*-tolyl)-1*H*-imidazol-5-yl]pyridine;
(iii) 4-[2-(naphthalen-2-yl)-4(5)-(*m*-tolyl)-1H-imidazol-5(4)-yl]pyridine;
(iv) 4-[2-(naphthalen-2-yl)-4(5)-(4-fluorophenyl)-1*H*-imidazol-5(4)-yl]pyridine;
(v) 4-[2-(1-bromonaphthalen-2-yl)-4(5)-(4-fluorophenyl)-1*H*-imidazol-5(4)-yl]pyridine;
(vi) 4-[2-(1-fluoronaphthalen-2-yl)-4-[3-(trifluoromethyl)phenyl]-1*H*-imidazol-5-yl]pyridine; and
(vii) 2-(1-fluoronaphthalen-2-yl)-4-(pyridin-4-yl)-5-(*m*-tolyl)oxazole.

In another preferred embodiment of the invention, the hematopoietic stem cells and progenitor cells are expanded in the presence of at least one cytokine selected from the group comprising stem cell factor (SCF), thrombopoietin (TPO), Fms-related tyrosine kinase 3 ligand (FLT-3L), interleukin 3 (IL-3), interleukin 6 (IL-6), granulocyte-colony stimulating factor (GCSF) and insulin-like growth factor binding protein 2 (IGFBP-2). More preferably the at least one cytokine is selected from the group comprising stem cell factor (SCF), thrombopoietin (TPO), Fms-related tyrosine kinase 3 ligand (FLT-3L) and insulin-like growth factor binding protein 2 (IGFBP-2). Preferably, the hematopoietic stem cells and progenitor cells are expanded in the presence of at least two, at least three or all four of SCF, TPO, FLT-3L and IGFBP-2. Preferably, the hematopoietic stem cells and progenitor cells are expanded in the presence of SCF, TPO, FLT-3L and IGFBP-2. More preferably, the hematopoietic stem cells and progenitor cells are expanded in the presence of 100 ng/ml SCF, 100 ng/ml TPO, 50ng/ml FLT-3L and 20 ng/ml IGFBP-2.

In another preferred embodiment of the invention, the method comprises culturing the umbilical cord blood mononuclear cell(s) with the at least one azole-based small molecule for a period of at least 9 days. Preferably, the method comprises culturing the umbilical cord blood mononuclear cell(s) with the at least one azole-based small molecule for a period of about 11 days. It would be understood that the period of culture may vary depending, for example, on the particular starting sample of umbilical cord blood, the growth rate of the cells or the number of cells required for grafting. It would be understood that bone marrow and/or mobilized peripheral blood, which also contain CD45+CD34+ HSPC cells may also be expanded according to the method of the invention.

In another preferred embodiment of the invention, the cytokines are added to the culture at day 0 and/or at day 7. The inventors found that day 7 was when the culture generally required the addition of fresh media due to cell expansion, so cytokines and azole-based small molecules were supplemented, if desired, at the same time. It would be understood that the requirement to replenish the media may vary around day 7, such as day 6 or day 8. The culture media may, for example, be supplemented with an equal volume of fresh media.

In another preferred embodiment of the invention, the at least one azole-based small molecule is added to the culture at day 0 and/or at day 7. It was found that the optimal expansion of cells occurred when the azole-based small molecules were added at day 0 and when the media was supplemented around day 7, although significant expansion was also obtained when the small molecules were added at time 0 only (for example, see Figure 12). It appears that if the small molecule is added at day 0, by about day 7 the number of cells produced causes the media to become exhausted and it needs to be supplemented to achieve optimal expansion.

In another preferred embodiment of the invention, the method further comprises the step of harvesting the cells after about 7 to 11 days in culture. Preferably, the cells are harvested around day 10 or 11 when optimal expansion is observed.

In another preferred embodiment of the invention, CD45+CD34+CD38-CD45RA-hematopoietic progenitor cells are expanded.

In another preferred embodiment of the invention, CD45+CD34+CD38-CD45RA-CD90+ (HSC1) hematopoietic stem cells are expanded.

In another preferred embodiment of the invention, CD45+CD34+CD38-CD45RA-CD90+CD49f+ (HSC2) hematopoietic stem cells are expanded.

In another preferred embodiment of the invention, the expanded hematopoietic stem and progenitor cells possess a normal karyotype and do not exhibit any signs of leukemic transformation.

In another preferred embodiment of the invention, a CD34- fraction of nucleated white blood cells is isolated and retained for use in co-transplantation with the *ex vivo* expanded cells into subjects in need thereof. It is understood that the CD34- fraction comprises lymphoid cells that may, if co-transplanted, reduce the likelihood of rejection or improve the engraftment of the transplanted *ex vivo*-expanded cells, particularly in humans.

In another aspect of the invention, the method further comprises a step of differentiating at least a proportion of the expanded hematopoietic progenitor cells and/or hematopoietic stem cells into NK cells. Such NK cells may be used to further treat cancer patients that have been treated with a graft of expanded hematopoietic progenitor cells and/or hematopoietic stem cells. The NK cells may be used in prophylaxis of patients at risk of relapse after treatment, or in treatment of patients that have relapsed after graft treatment.

In another aspect of the invention, there is provided a combination and/or kit comprising at least one azole-based small molecule according to any aspect of the invention; and at least one cytokine.

In a preferred embodiment of the combination and/or kit, the at least one cytokine is selected from the group comprising SCF, TPO, FLT-3L and IGFBP-2 for use in *ex vivo* expansion of the hematopoietic stem cells and progenitor cells component of umbilical cord blood.

In another preferred embodiment, the at least one azole-based small molecule expands CD45+CD34+CD38-CD45RA-CD90+ hematopoietic stem cells and/or CD45+CD34+CD38-CD45RA-CD90+CD49f+ hematopoietic stem cells and/or CD45+CD34+ CD38-CD45RA- hematopoietic progenitor cells.

In another aspect of the invention, there is provided a composition comprising at least one azole-based small molecule defined according to any aspect of the invention for use in *ex vivo* expansion of the hematopoietic stem cells and progenitor cells component of umbilical cord blood. It would be understood that bone marrow and/or mobilized peripheral blood, which also contain CD45+CD34+ HSPC cells may also be expanded by the compounds of the invention.

Cells obtained by a method according to any embodiment of the invention may be used in the manufacture of a medicament for the treatment of a disease requiring hematopoietic stem cell transplantation.

The medicament may comprise the *ex vivo* expanded cells and the retained CD34-lymphoid cells.

In another aspect of the invention, there is provided a use of at least one azole-based small molecule as herein defined, in *ex vivo* expansion of the hematopoietic stem cells and progenitor cells component of umbilical cord blood. It would be understood that bone marrow and/or mobilized peripheral blood, which also contain CD45+CD34+ HSPC may also be expanded by the compounds of the invention.

In a preferred embodiment the at least one azole-based small molecule expands CD45+CD34+CD38-CD45RA-CD90+ hematopoietic stem cells and/or CD45+CD34+CD38-CD45RA-CD90+CD49f+ hematopoietic stem cells and/or CD45+CD34+ hematopoietic progenitor cells.

The at least one azole-based small molecule as herein defined may be used for the manufacture of a medicament for the prophylaxis or treatment of a patient in need of expansion of their CD45+CD34+CD38-CD45RA-CD90+ hematopoietic stem cells and/or CD45+CD34+CD38-CD45RA-CD90+CD49f+ hematopoietic stem cells and/or CD45+CD34+CD38-CD45RA- hematopoietic progenitor cells.

A method of treatment may comprise administering to a subject in need of such treatment an efficacious amount of hematopoietic stem cells and progenitor cells obtained by a method according to any aspect of the invention. In a preferred embodiment, the treatment comprises also administering an efficacious amount of CD34- lymphoid cells to the subject.

A method of treatment may comprise administering to a subject in need of such treatment an efficacious amount of an azole-based small molecule according to any aspect of the invention. The method may, for example, comprise intravenous administration. Patients in need of such treatment may have a low blood cell count (post-chemotherapy or total body irradiation) or a bone marrow disease.

The subject may have a hematopoietic disorder selected from Acute myeloid leukemia, Acute lymphoblastic leukemia, Chronic myeloid leukemia, Chronic lymphocytic leukemia, Myeloproliferative disorders, Myelodysplastic syndromes, Multiple myeloma, Non-Hodgkin lymphoma, Hodgkin's disease, Aplastic anaemia, Pure red cell aplasia, Paroxysmal nocturnal hemoglobinuria, Fanconi anemia, Thalassemia major, Sickle cell anaemia, Severe combined immunodeficiency, Wiskott-Aldrich syndrome, Hemophagocytic lymphohistiocytosis and inborn errors of metabolism.

### EXAMPLE 1

### METHODS

### UCB collection, processing, thawing and plating

UCB was obtained through Singapore Cord Blood Bank (SCBB), from donated units failing to meet the criteria for clinical banking. Prior consent was obtained from the donating mothers and the Research Advisory Ethics Committee of the SCBB, along with the Institutional Review Boards of National University of Singapore (NUS), and Singapore General Hospital (SGH) approved the usage of the samples. Mononuclear cells (MNC) were isolated from the fresh UCB by density gradient centrifugation using Ficoll-Histopaque^{™} Premium (GE Healthcare, UK). Counted UCB-MNC was cryopreserved in 90% v/v autologous plasma with 10% v/v dimethyl-sulfoxide (DMSO) (Sigma Aldrich, USA) for subsequent usage. A brief summary of the method is shown in Figure 3. UCB-MNC was thawed using human serum albumin (25% v/v) (Health Sciences Authority, Singapore) and Dextran 40 (75% v/v) (Hospira, USA). UCB-MNC were cultured at an empirically determined optimal density of 4.0×10⁵ cells/mL without any cell surface marker dependent stem cell enrichment in StemSpan^{™} Serum-Free Expansion Media (SFEM) or Animal Component Free Media (ACF) (STEMCELL Technologies, Canada) supplemented with human cytokine cocktail of 100 ng/mL stem cell factor (SCF) (PeproTech, USA) and thrombopoietin (TPO) (PeproTech, USA); 50 ng/mL FLT-3 Ligand (FLT-3L) (PeproTech, USA); and 20 ng/mL insulin-like growth factor binding protein-2 (IGFBP-2) (R&D Systems, USA). Various cytokine combinations (at the respective concentrations described supra) were tested on UCB-MNC with and without 5 µM IM-29 to determine their effects on expansion of TNC and HSPC over 11 days in ACF media. For certain experiments, cell cultures were initiated with purified populations such as early (CD45+CD34+CD38-) or late (CD45+CD34+CD38+) progenitor cells at optimal plating concentration of 5.0×10⁴ cells/ml or 2.0×10⁶ cells/ml, respectively. Such pure populations were obtained by labeling the frozen-thawed, non-enriched UCB-MNC using fluorescence conjugated antibodies followed by fluorescence activated cell sorting (FACS). The substituted azole-based small molecules dissolved in DMSO were added to the cultures at an empirically determined optimal concentration of 5.0 µM. UCB-MNC cultures devoid of small molecules but supplemented with cytokines served as control, while cultures supplemented with cytokines and DMSO served as vehicle control.

Cell cultures for *in vitro* experiments were done in 6- or 24-well plates (BD Falcon, USA), while culturing for *in vivo* transplantation studies was carried out in T-175 flasks (Corning, USA). Cell cultures were maintained in a humidified, 5% carbon dioxide incubator at 37°C for the required duration. For UCB expansion evaluation and animal experimentation, an established 10-11-day expansion protocol was used which included cytokine and small molecule replenishment on day 7. At completion of incubation, cells were aspirated from the culture-ware with subsequent rinsing by Dulbecco's phosphate buffered saline (DPBS) (Hyclone, USA). The extracted cells were counted using an automated differential hematology cell counter (COULTER^{®} AcT^{™} diff Hematology Analyzer, Beckman Coulter Inc, USA) and re-suspended in DPBS for subsequent *in vitro* analysis or transplantation in mice.

### Colony forming unit assays

Colony-forming units (CFU) of granulocyte-monocyte (GM) from freshly thawed UCB-MNC or 11 days expanded cells of the mentioned cell cultures were evaluated. Duplicates of freshly thawed UCB-MNC (5,000 and 10,000 cells) and expanded cells (1,000 and 5,000 cells) were cultured in 35 mm petri dishes (BD Falcon, USA) in 1.1 mL of hematopoietic stem cell (HSC)-CFU complete media with erythropoietin (EPO) (Miltenyi Biotec, Germany) without any further media manipulation. After 14-16 days in culture in a humidified environment at 37°C and 5% CO₂, colonies were scored and pictured using a SZ61 Olympus microscope equipped with charge-coupled device (CCD) camera (Olympus Europa GmbH, Germany).

### Animal maintenance, transplantation and procedures

The xenotransplantation studies were approved by the Singapore Health Services (SingHealth) Institutional Animal Care and Use Committee. NOD.Cg-Prkdc^{scid} II2rg^{tm1Wjl}/SzJ, better known as non-obese diabetic (NOD) - severe combined immunodeficient (SCID) gamma (NSG) mice, purchased from Jackson Laboratory (Bar Harbor, USA), were housed in cages of six of the same gender in SingHealth Experimental Medicine Centre. Sterilized food and water were accessible *ad libitum.* Following acclimation and successful breeding, the sub-lethally irradiated (240 cGy) 8 - 12 weeks old mice were randomly divided into five experimental groups for tail vein administration of: (i) saline; (ii) non-expanded UCB-MNC; (iii) cytokine expanded UCB-MNC in StemSpan^{™}-SFEM or StemSpan^{™}-ACF (control expansion cultures); (iv) IM-29 and cytokine expanded UCB-MNC in StemSpan^{™}-SFEM or StemSpan^{™}-ACF. To investigate the *in vivo* human cell engraftment kinetics, expanded UCB (± IM-29 in SFEM or ACF) were transplanted at an empirically optimized equivalent dosage of 2.5×10⁷ cells/kg, 5.0×10⁷ cells/kg or 10.0×10⁷ cells/kg while non-expanded UCB was transplanted at an absolute dosage of 2.5×10⁷ cells/kg, 5.0×10⁷ cells/kg or 10.0×10⁷ cells/kg. Magnetic antibody-labelled and column (Miltenyi Biotec, Germany) purified (as per manufacturer's protocol) human CD45⁺ cells obtained from the bone marrow of *primary* NSG recipients after 20 weeks of transplantation were administered to *secondary* NSG recipients *via* tail vein injection for the following experimental groups: (i) non-expanded UCB-MNC; (ii) cytokine expanded UCB-MNC and (iii) IM-29 and cytokine expanded UCB-MNC at transplantation doses of 1×10⁶ - 2×10⁶ cells/mouse.

All mice received antibiotics and immunosuppressive drugs to minimize bacterial infection and reduce chances of graft-versus-host-disease (GVHD), respectively. Briefly, for all experimental groups, Cyclosporine (Novartis, USA) immunosuppressive therapy started the day after the experimental cell inoculation at a dosage of 10 mg/kg for the first two consecutive days and then 15 mg/kg on every other day for three more doses (five doses in total). Acidified (pH=2.2) drinking water containing 1.1 g/L of neomycin trisulfate (Sigma-Aldrich, USA) and 0.1 g/L of polymycin B sulphate (Sigma-Aldrich, USA) was given for 7 days pre-transplantation and another 23 days post-transplantation to minimize bacterial infection. Assessment of human cell reconstitution after two - three weeks of transplantation was done using blood samples collected *via* the submandibular vein. The mice were sacrificed at end of week 2 or 20 to harvest the bone marrow to analyze human multi-lineage reconstitution.

### Flow cytometric analysis and cell sorting

All data were acquired using the Cytomics FC500 Flow Cytometer (Beckman Coulter, Inc., USA) or BD^{™} LSR II (Becton Dickinson, USA) at least 10,000 events per sample were collected. Acquired data were subsequently analyzed with CXP Analysis Software (Beckman Coulter, Inc., USA) or BD FACSDiva^{™} 8.0 Software (Beckton Dickson, USA). BD FACSAria^{™} III (Beckton Dickson, USA) was used for sorting out the early (CD45+CD34+CD38-) or late (CD45+CD34+CD38+) progenitors from UCB-MNC that were labeled with the appropriate sterile fluorescence conjugated monoclonal antibodies (Figure 17G) described below. Titration was performed to identify optimal antibody staining. Isotype controls were used for the purposes of gating out non-specific antibody binding during analysis.

Phycoerythrin (PE) conjugated CD34, allophycocyanin conjugated (APC) CD38 and phycoerythrin-Cy7 (PE-Cy7) conjugated CD45, were used for phenotypic analysis or sterile sorting of the hematopoietic progenitor cells (HPC). CD45RA-V450, CD90-FITC (fluorescein isothiocyanate) and CD49f-PerCP-Cy5.5 were used in combination with HPC antibodies to probe rare HSPC populations. Lymphoid lineage progenitors and differentiated cells were phenotyped using CD7-FITC, CD3-BV605, CD19-BUV395, CD56-V450 and CD138-PerCP-Cy5.5. Myeloid lineage progenitors and differentiated cells were phenotyped using CD33-PE-Cy7, CD41a-FITC, CD15-BUV395, CD13-BV421 and CD61-PerCP-Cy5.5. In all these phenotypic expression studies, live and dead cells were distinguished using 7-Aminoactinomycin D (7-AAD). All antibodies were bought from BD Pharmingen (USA).

Annexin-V-FITC (Beckman Coulter, Inc., USA), 7-AAD (Beckman Coulter, Inc., USA) and CD45-PE-Cy7 were used for CD45+ cell viability analysis.

Analysis of human chimerism in the mice peripheral blood was carried out at day 14, 21, 42, 63, 84, 105, 126 or 196 post-transplantation of the non-expanded and expanded grafts. Approximately 190 µl of each blood sample underwent ammonium chloride (in-house formulation) dependent red blood cell lysis followed by blocking using mouse and human FcR reagents to minimize non-specific antibody binding. The remaining white blood cells in the samples were stained with anti-human CD45-APC, CD3-PE/FITC, CD19-VioBlue/PE-Vio615, CD33-PE-Vio770, CD15-PerCP-Vio770, CD34-PE and anti-mouse CD45-FITC/VioGreen. All antibodies and blocking reagents were bought from Miltenyi Biotec (Germany).

The bone marrow of an individual mouse was flushed out from both femurs and tibias using 2% fetal bovine serum (FBS) (Sigma-Aldrich, USA) supplemented RPMI media (Invitrogen, USA) at week 2 or 20 post-transplantation. Ammonium chloride was used to lyse the red blood cells (RBC) in all samples. DPBS (Hyclone, USA) with 2% FBS (Sigma-Aldrich, USA) was used to wash out and re-suspend the nucleated cells for further human cell surface marker/antigen analysis using appropriate fluorescent conjugated antibodies and flow cytometer. Briefly, the remaining white blood cells in the bone marrow samples were stained with anti-human CD45-APC and anti-mouse CD45-FITC/VioGreen to differentiate human and mouse cells. Human CD34-PE was used to analyze human progenitor cells. Human myeloid cells were analyzed by staining with CD71-VioBlue, CD33-PE-Vio770, CD15-PerCP-Vio770, CD13-PE-Vio615, CD66b-APC-Vio770 and CD41a-VioGreen. Human lymphoid cells were analyzed by staining with CD3-VioGreen, CD4-VioBlue, CD7-APC-Vio770, CD8-PerCP-Vio700, CD19-PE-Vio615 and CD56-PE-Vio770. All antibodies and blocking reagents were bought from Miltenyi Biotec (Germany).

Upon completion of antibody staining, all labeled cells were washed with DPBS (Hyclone, USA) and subsequently re-suspended in DPBS (Hyclone, USA) with 2% FBS (Sigma-Aldrich, USA) for flow cytometer based analysis.

### Fluorescence in situ hybridization (FISH)

UCB-MNC samples were fixed with modified Carnoy's fixative (Leica Biosystems, Germany) and placed onto glass microscope slides, and then dehydrated through an ethanol (Sigma-Aldrich, USA) series (70%, 85% and 100%) for 2 minutes followed by air-drying.

FISH assays were carried out using a panel of probes (Abbott Molecular, USA) comprising LSI D7S486 SpectrumOrange^{™}/CEP 7 SpectrumGreen^{™}, CEP 8 SpectrumAqua^{™}/LSI MYC SpectrumOrange^{™}, LSI CDKN2A SpectrumOrange^{™}/CEP 9 SpectrumGreen^{™}, LSI ABL1 SpectrumOrange^{™}/BCR SpectrumGreen^{™} dual fusion translocation probe, LSI MLL dual color break-apart probe, LSI ETV6 SpectrumGreen^{™}/RUNX1 SpectrumOrange^{™} extra signal dual color translocation probe, and LSI TP53 SpectrumOrange^{™}/CEP 17 SpectrumGreen^{™} probe set. The FISH probes were applied to the fixed cells and co-denatured at 75°C, followed by an overnight hybridization at 37°C. Washes were performed and the slide was counterstained with DAPI anti-fade solution (Vectashield, Vector Laboratories, USA) and analyzed using an epi-fluorescence microscope (Leica, Germany).

Signals from 100 non-overlapping nuclei were enumerated for loss of LSI D7S486, trisomy 8, loss of CDKN2A, translocation involving ABL1 and BCR, MLL break-apart, translocation involving ETV6 and RUNX1, and loss of TP53. A normal signal pattern is defined as two copies of D7S486 and CEP7, two copies of CEP 8 and MYC, two copies of CDKN2A and CEP 9, absence of ABL1/BCR fusion signals, intact MLL dual fusion signals, absence of ETV6/RUNX1 fusion signal, and two copies of TP53.

### Cytogenetics/Karyotyping

Non-cultured UCB-MNC and Day 10 cultured cells in StemSpan^{™}-ACF containing standard cytokines cocktail in presence or absence of the lead compound IM-29 were used for karyotyping. To study the karyotype the UCB-MNC cells were further cultured for 48 hours in a humidified 5% CO₂ incubator maintained at 37°C using RPMI 1640 media (Gibco, USA) supplemented with fetal calf serum (Sigma, USA), L-glutamine (Gibco, USA) and antibiotics (Gibco, USA). The cultures were then harvested and G-banded according to standard clinical laboratory protocol. Twenty cells were analyzed and the karyotype was described in accordance to the International System for Human Cytogenetic Nomenclature (2016).

### Leukocyte cytochemistry

Cell smears from freshly thawed or cultured UCB-MNC cells (± 5.0 µM) were stained with May-Grünwald Giemsa (MGG), Sudan Black B, Periodic acid-Schiff (PAS) and myeloperoxidase stain (p-phenylenediamine and catechol) using standard clinical laboratory protocols and imaged using an upright microscope. All stains were obtained from Sigma-Aldrich, USA.

### Statistical analysis

Results are reported as mean ± standard error of the mean (SEM) or mean ± standard deviation (SD) for the specified n value stated in the brief description of the figures. The significance of difference between two groups was determined using the 2-tailed Student t-test and the *P* value is stated in the brief description of the figures. Data processing and statistical analyses were performed with OriginPro^{®} 9.1 (OriginPro, USA), GraphPad Prism 6.0 (GraphPad Software, Inc., USA) and Microsoft Office Excel (Microsoft, USA).

### EXAMPLE 2

### Major method steps

In an example of the invention, the major steps involved in the method of expanding HSPC from frozen-thawed UCB-MNC using IM-29 are shown in Figure 2:
(i) Process fresh UCB using density dependent centrifugation to isolate mononucleated (MNC) fraction which is frozen down at -180°C for future expansion;
(ii) Thaw and culture UCB-MNC in defined culture medium containing a cytokine cocktail of SCF, TPO, FLT-3L and IGFBP-2;
(iii) Add IM-29 at a final concentration of 5.0 µM;
(iv) Incubate cells in a humidified incubator maintained at 37°C and 5% CO₂;
(v) At day 3 - monitor the viability of the leukocyte cells (WBC) that express CD45. HSPC is a subset of CD45 cells;
(vi) At day 7 - replenish (top-up) growth media, cytokines and IM-29;
(vii) At day 10/11 - harvest cells for assessing expansion using *in vitro* phenotypic and functional assay and *in vivo* transplantation to immunodeficient mice to monitor repopulation capacity.

The changes in cell composition during expansion are shown in Figure 5.

### EXAMPLE 3

### Small molecules derived from compound SB203580

The small molecule library consisted of several analogues, all of which were derived from the parent compound SB203580 (Figure 6D) which is a known inhibitor of p38 MAPK (mitogen activated protein kinase), with optimal activity at a working concentration of 5 to 10 µM. Compound IM-29 with chemical structure shown in Figure 6A is the most effective of those tested. IM-04 with chemical structure shown in Figure 6B is the second most effective compound. Structural analogues of IM-29 and IM-04 that gave sub-optimal effect are shown in Figure 6C. A total of forty analogues of SB203580 were generated for this study, which are shown in Figure 6E and broadly divided into four groups based on the structure and chemical modification. Group 1 of Figure 6E examined the variation of the substituents at C-2 position of imidazole while retaining the vicinal pyridine-4-yl/3-tolyl or pyridine-4-yl/3-(trifluoromethyl)phenyl moiety at C-4 and C-5 positions of the imidazole which gave rise to a total of six analogues. The second best compound IM-04 is a member of Group 1. A further thirteen different analogues were generated in Group 2 (as shown in Figure 6E), where the pyridine-4-yl substituent at C-5 position was replaced with a pyran-4-yl substituent while retaining the tolyl-group or 4-fluorophenyl substituent at C-4 position of the imidazole. The structure of the compounds in Group 3 of Figure 6E was used to investigate the variation in the substituents at C-2 position of imidazole while retaining the vicinal pyridine-4-yl/4-fluorophenyl moiety. The lead compound IM-29 is a member of Group 3. In Group 4 (Figure 6E) the imidazole core structure was replaced by oxazole. Structure-activity relationship studies were carried out based on all the analogues shown in Figure 6E to identify specific chemical structures and modifications that were critical in mediating HSPC expansion. Generally it was observed that imidazoles with the vicinal pyridine-4-yl/4-fluorophenyl substituents that can provide the aromatic region and H-bond acceptor at the C5 and C4 positions exhibited higher activities in inducing *ex vivo* expansion of HPCs. If the substituent at C4 of imidazole was replaced with tolyl or 3-(trifluoromethyl)phenyl group it decreased the analogues' ability to augment HPC expansion. Similarly, if the substituent at C5 of imidazole was replaced with pyran-4-yl group it significantly reduced the HPC expansion. The best substituent for the C2 position of azoles is the naphthyl substituent, and of these, the compound IM-29 which has a 1-fluoronaphthalen-2-yl substituent was identified to be the most potent compound for the induction of *ex vivo* expansion of HPCs among all the compounds screened. Replacing 1-fluoronaphthalen-2-yl of IM-29 at C2 position of the imidazole with naphthalen-2-yl (such as in the compound ZQX-33: 4-[2-(naphthalen-2-yl)-4(5)-(4-fluorophenyl)-1*H*-imidazol-5(4)-yl]pyridine) reduced the HPC expansion capacity by at least 2-fold (*P* < 0.001). Finally, the oxazole compound (OZ-07) was not optimally active in inducing *ex vivo* expansion of HPC, suggesting that it is essential to have a H-bond donating group at the central structure of the molecule.

All compounds were assessed for their ability to maintain the viability of CD45+ leukocytes using Annexin V and 7-AAD. Induction of apoptosis in the CD45+ cells during *ex vivo* cultures limits expansion of HSPC. All compounds demonstrated minimal acute toxicity to the UCB cells (Figure 7).

### EXAMPLE 4

### Analogue IM-29 significantly improves HPC expansion ex vivo

IM-29 at a concentration of 5.0 µM was shown to expand hematopoietic progenitor cells (HPC) with the expression profile CD45+CD34+CD38-CD45RA- by at least 1,200-fold over 10 days (Figure 8A). Compared to cytokine control, IM-29 could impart an enhancement effect of 8-fold for HPC expansion. IM-04 expanded HPC between 1,000 to 1,150-fold over 10 days (Figure 8A), whereas IM-01, ZQX-33, ZQX-36, GJ-C and OZ-07 expanded HPC between 400 to 900-fold over 10 days (Figure 8A). The screen was repeated in animal-component-free (ACF) media, including additional azole-based small molecules, and expansion data are presented in Figure 8B. In addition, IM-29 increased HPC-associated expression of CD45+CD34+CD38-CD45RA- to about 68% which was 3-fold higher than cytokine control (Figure 8C).

Prior experiments were carried out with small molecules being supplemented at 5.0 µM since it is the optimal working concentration for the parent compound SB203580; however, it was necessary to identify the optimal working concentration of IM-29 in expanding HPC. As shown in Figure 9A, optimal expansion of TNC was achieved with an IM-29 concentration of 5.0 µM for cultures initiated with non-selected MNC. At 1.0 µM or 10 µM of IM-29 the expansion of total nucleated cells (TNC) was reduced by 0.83-fold and 0.70-fold, respectively, compared to 5.0 µM. Similarly, 1.0 µM, 5.0 µM and 10.0 µM of IM-29 could enhance expansion of HPC by 2.7-fold, 3.6-fold and 2.4-fold, respectively, compared to cytokine control (Figure 9A). All subsequent experiments were carried out using IM-29 at a working concentration of 5.0 µM.

As IM-29 is a novel small molecule that expands HPC, we investigated the effect of this compound when it was supplemented to culture with varying combination of cytokines with the aim of identifying an optimal cytokine combination (Figure 1). Cytokines are critical in expanding HSPC with the most commonly used cocktail consisting of SCF, TPO and FLT-3L. As shown in Figure 1, an optimal HPC expansion of 1513.9±6.4-fold was observed when cultures were supplemented with SCF (S), TPO (T), FLT-3L (F), IGFBP-2 (IG) and IM-29 (IM) which was at least 4.7-fold higher than the four cytokines (S+T+F+I) culture (P < 0.05). When the basal cytokine cocktail consisted of only three cytokines (such as combinations of S+T+F; T+F+IG; F+IG+S; and S+IG+T), the addition of IM-29 could significantly (P < 0.05) boost expansion of HPC. For example, comparing the combinations of S+T+F (486.8±27.2-fold) and S+T+F+IM-29 (1265.2±39.1-fold) we observed an expansion enhancement effect of 2.6-fold (*P* < 0.05). Similarly, a two-fold augmentation of HPC expansion is observed when IM-29 is added to a basal cocktail of S+IG+T (P < 0.05). Interestingly, IM-29 could support comparatively better expansion of HPC even when only two cytokines (example S+T or T+F) were added to the culture system. However, minimal expansion was observed if IM-29 was used with certain combinations of cytokines (for example S+IM; T+IM; F+IM or IG+IM) (data not shown). The addition of IM-29 alone (i.e. without any cytokines) did not support the expansion of HPC, which was similar to growing the cells devoid of any growth factors (data not shown). In terms of TNC, optimal expansion was observed when the cytokine cocktail consisted of S+T+F+IG along with IM-29 (Figure 1).

IM-29 treated cells could enhance the expansion of colony forming units (CFU) by at least 100-fold compared to non-cultured cells, whereas expansion with cytokines alone resulted in about 25-fold increase in CFU compared to the non-cultured fraction (Figure 9B).

The addition of IM-29 to either serum-free expansion media (SFEM that contains bovine serum albumin) or animal component free (ACF which is chemically defined) media allowed significantly better expansion of UCB HPC as measured by phenotypic and functional assay (Figure 10). IM-29 increased the expansion of HPC by at least 2 to 3-fold compared to cytokine control. In terms of CFU, addition of IM-29 increased granulocyte, monocyte (GM) colonies by 2.5 to 5-fold compared to cytokine control. The data suggests that IM-29 could work with different basal media for expansion.

### EXAMPLE 5

### Effect of IM-29 timing and time in culture on expansion of HPC

Increasing the culturing period of UCB in IM-29 supplemented cultures from 7 days to 9 days boosted the expansion of HPC by at least 5-fold. However, in cytokine cultures the increase in HPC was only 2.7 fold over the same time period. By day 11, IM-29 increased the total nucleated cells (TNC) by about 6-fold compared to starting cell number, whereas cytokine controls increased TNC by at most 3-fold (Figure 11).

Adding IM-29, at both day 0 and day 7, enhanced expansion of HPC (CD45+CD34+CD38-CD45RA-) by at least 750- and 450-fold in serum-free expansion media (SFEM) and animal-component-free (ACF) media, respectively, over 10 days (Figure 12, Group 1). Irrespective of basal culture media, the HPC expansion of Group 1 was at least 12-fold higher than the cytokine control Group 3. In Group 2, when IM-29 was not replenished on day 7, the expansion of HPC was reduced by at least 0.7-fold compared to Group 1. Addition of IM-29 only at day 7, i.e. it is not added at start of culture (Group 4), had negligible effect on expanding HSPC. Therefore, it is necessary to add IM-29 at both day 0 and 7 to enable optimal expansion of UCB HSPC.

### EXAMPLE 6

### IM-29 increases the proportion of immunodeficient mice engrafting cells HSC1 and HSC2

In presence of IM-29 and cytokines, the percentage expression of both CD45+CD34+CD38-CD45RA-CD90+ (HSC1) and CD45+CD34+CD38-CD45RA-CD90+CD49f+ (HSC2) increased by 4 to 5-fold compared to non-cultured cells (Figure 13A). In terms of absolute cell numbers, IM-29 increased the proportion of immunodeficient mice engrafting cells (HSC1: CD45+CD34+CD38-CD45RA-CD90+) to at least 1,000-fold over 10 days compared to day 0, whereas cytokine-only controls could merely increase the same population by about 80-fold (Figure 13B). In terms of HSC2 defined by CD45+CD34+CD38-CD45RA-CD90+CD49f+, IM-29 could enhance expansion by at least 7.5-fold compared to cytokine-only control over 10 days (Figure 13C).

### EXAMPLE 7

### IM-29 cultured cells maintain normal karyotype

Cytogenetic analysis revealed that IM-29 cultured cells maintained normal karyotype (Figure 13D) showing no differences when compared with karyotypes of non-cultured cells (data not shown). Fluorescence *in situ* hybridization (FISH) using various probes relating to hematological malignancies revealed normal results for IM-29 expanded grafts (Figure 13E) compared to cytokine expanded grafts or non-cultured grafts (data not shown). Cell morphology and leukocyte cytochemistry analysis showed no evidence of leukemic transformation of the IM-29 expanded grafts (Figure 13E).

A schematic describing the method of transplanting IM-29 expanded UCB grafts into an immunodeficient mouse model is shown in Figure 14. Engraftment data obtained from transplanting UCB mononuclear cells that were expanded with IM-29 is shown in Figure 15 and Figure 16. Transplantation of IM-29 expanded UCB grafts (n=11) at equivalent dosage of 2.5×10⁷ cells/kg to sub-lethally irradiated NOD SCID Gamma (NSG) mice resulted in 3.53- and 2.09-fold higher engraftment of human CD45+ cells in the peripheral blood by day 21 compared to non-expanded (P=0.0030; n=11) and cytokine expanded grafts (P=0.0005; n=12), respectively (Figure 15A). Freeze-thaw of the expanded grafts prior to transplantation into the NSG mice showed that IM-29 graft maintained *in vivo* repopulation capacity (*P* = 0.0730 between fresh and frozen-thawed IM-29 expanded graft; Figure 15A), whereas cytokine expanded graft had reduced engraftment of human CD45 cells in the peripheral blood (PB) of NSG mice at week 3 (*P* = 0.0008 between fresh and frozen-thawed cytokine expanded graft; Figure 15A). The IM-29 expanded graft sustained human cell engraftment in the PB of the NSG mice for up to at least 19 weeks (data not shown). The graft comprised primarily myeloid cells (CD33+/CD15+), as opposed to non-expanded graft which consisted of CD3+ T cells (Figure 15B). Moreover, IM-29 expanded grafts allowed quick engraftment of donors' cells. The frequency of SCID repopulating cells contributing to early peripheral blood engraftment was 2.48-fold higher in IM-29 expanded graft compared to unmanipulated graft.

### EXAMPLE 8

### IM-29 expanded grafts impart long term hematopoiesis in NSG mice

IM-29 expanded grafts retained the ability to impart long-term hematopoiesis as observed by analyzing the bone marrow of recipient NSG mice at 19 weeks post-transplantation (Figs. 16A-16E). As has been reported by others [Notta F, et al., Blood 115(18): 3704-7 (2010); McDermott SP, et al., Blood 116(2):193-200 (2010)], in this mouse model irrespective of graft (i.e. expanded or non-expanded), female recipients had higher engraftment rates than their male counterparts (Figure 16A). Despite a difference in absolute geometric means, the IM-29-expanded grafts gave a statistically comparable level of human CD45 (Figure 16B) and common (CD45+CD34+), myeloid (CD45+CD13+CD33+) and lymphoid (CD45+CD7+) progenitor cell engraftment as that of the non-expanded grafts (Figure 16C) at transplantation dosage of 2.5 × 10⁷ cells/kg and 5.0 × 10⁷ cells/kg in both male and female recipients. Furthermore, similar to early engraftment of human CD45 cells in PB (Figure 15A), the administration of frozen-thawed IM-29 expanded grafts maintained comparable long-term bone marrow (BM) human cell engraftment (*P* = 0.6593 between fresh and frozen-thawed IM-29 expanded graft; Figure 16B). Multi-lineage reconstitution of NSG BM comprising both mature myeloid (Figure 16D) and lymphoid (Figure 16E) human cells could be achieved with the IM-29-expanded graft although initial peripheral blood engraftment was skewed towards the myeloid lineage. Furthermore, the IM-29 expanded grafts did not exhibit any leukemic transformation in the transplanted NSG mice bone marrow (BM).

### EXAMPLE 9

### IM-29 and cytokine supplemented cultures primarily maintain and increase myeloid lineage mature cells from UCB MNC

The data shown in Figure 17A indicates that IM-29 and cytokine supplemented cultures primarily maintain and increase myeloid lineage mature cells (which consists of CD45+CD33+ monocytes, CD45+CD13+CD15+ granulocytes and CD45+CD41a+CD61+ megakaryocytes) when *ex vivo* expansion cultures are initiated with mono-nucleated cells (MNC) of the UCB. This means that the IM-29 expanded graft is devoid of mature lymphoid cells (which consists of CD45+CD3+ T cells, CD45+CD19+ B cells and CD45⁺CD56⁺ NK cells) prior to transplantation. As shown in Figure 17B, at a higher transplantation dosage of 100 million cells/kg, IM-29 expanded grafts produced 7.1 ± 0.6 % of human CD45⁺ cells in the NSG PB by week 2 which was at least 5-fold higher than cytokine expanded graft recipients (P < 0.0001; n = 14) further supported by an absolute increase in total human cell number. However, at such a high transplantation cell dose of 100 million cells/kg, non-expanded UCB gave significantly (*P* < 0.0001; n = 15) higher engraftment by at least 3.7-fold compared to an IM-29 expanded graft (Figure 17B). Similar to the data shown in Figure 15B, non-expanded grafts at higher cell dosage transplants gave rise to primarily CD3+ T cells in the NSG PB at week 2 post-transplantation, while IM-29 expanded grafts maintained minimal human T cell populations (Figure 17C). Analysis of the NSG mice bone marrow (BM) at week 2 post-transplantation, showed that non-expanded (n = 6) and IM-29 expanded grafts (n = 6) reproduced similar human CD45+ cell engraftment which was significantly (*P* < 0.01) higher than cytokine expanded control grafts (n = 6) (Figure 17D). In terms of CD34+ human progenitors in the NSG BM, IM-29 expanded grafts maintained 13.3 ± 0.8% (n = 6) compared to 0.7 ± 0.1 % of non-expanded grafts (n = 6) at week 2 post-transplantation (*P* < 0.001) (Figure 17D). Similar to the PB engraftment data, NSG mice transplanted with non-expanded UCB had a predominant proportion of CD3+ T cells in the BM compared to expanded grafts (Figure 17D). However, it must be noted based on data shown in Figures 16A-16E, that although IM-29 expanded graft skews early human cell engraftment towards progenitors and myeloid cells, in the long-term studies (>19 weeks post-transplantation) it also gives rise to lymphoid cells in the BM of the NSG mice thus maintaining multi-lineage reconstitution. The increased amount of human T cells reconstituted from the non-expanded graft resulted in higher incidence of graft-versus-host-disease (GVHD) in the NSG mice recipients which resulted in poorer survival rate of approximately 25% at day 60 post-transplantation (Figure 17E). Survival of the NSG mice receiving the expanded grafts (with or without IM-29) had > 70% survival at day 60 post-transplantation due to minimal symptoms of GVHD (Figure 17D).

When the efficacy of IM-29 expanded grafts is to be studied in a phase I clinical trial, it will be necessary to infuse a second non-manipulated graft as a measure of clinical safety. Based on the data shown in Figures 17A-17H, it is evident that expansion of UCB MNC in the presence of IM-29 primarily gives rise to CD34+ progenitors and mature myeloid cells. Such an expanded graft devoid of its lymphoid cells if co-infused with a second immune cell-containing non-manipulated graft (UCB2, Fig. 17F) would be likely to face immune-rejection that would result in graft failure. Therefore, in a phase I clinical trial it will be necessary to infuse CD34- lymphoid cells cryopreserved during the CD34 selection of UCB1 graft along with the non-manipulated second unit (UCB2). This could be achieved by the following steps depicted in the schematic of Figure 17F:
(i) Step 1 - Obtain clinical frozen UCB unit 1 (UCB1) that has insufficient cell dosage for transplant. Perform thawing, washing and magnetic column based CD34+ selection of the unit.
(ii) Step 2 - Culture the CD34+ cells of UCB1 in an IM-29 expansion protocol as described above.
(iii) Step 3- Cryopreserve the CD34-fraction of UCB1 which contains the lymphoid lineage mature cells.
(iv) Step 4 - Expand UCB1 CD34+ cells for 10 - 11 days with media, cytokine and IM-29 replenishment at day 7.
(v) Step 5 - Harvest, wash and characterize expanded UCB1.
(vi) Step 6 - Infuse the expanded portion of the UCB1 into the patient.
(vii) Step 7 - Thaw, wash and infuse the CD34- fraction of UCB1 into the patient.
(viii) Step 8 - Obtain clinical frozen UCB unit 2 (UCB2) that has sufficient cell dosage for transplant. Perform thawing, washing and infusion into the patient.

Although IM-29 was able to expand HSPC from non-enriched UCB MNC, it was necessary to study the expansion effect of this molecule when cultures were initiated with purified CD34+ cells to support phase I clinical trial expansions. In cultures initiated with purified CD34+CD38- cells (using fluorescence conjugated antibody labeling following by fluorescence activated cell sorting), there was at least 15.9-fold higher expansion of HSC1 defined by CD45+CD34+CD38-CD45RA-CD90+ in presence of IM-29 compared to cytokine cultures (*P* < 0.0001) (Figure 17G). Finally, UCB grafts were enriched for CD34 cells using magnetic columns to mimic clinical grade selection methods. Culturing of these CD34⁺ cells in presence of 5.0 µM IM-29 and cytokine cocktail (SCF, TPO, FLT-3L and IGFBP-2) resulted in 283.7 ± 14.7-fold of CD34+ cells within 11 days which was approximately 1.9-fold higher compared to cytokine control cultures (Figure 17H).

### Comparison with other known methods

Similar HSPC enriched cultures with competing small molecule stemregenin-1 (SR-1) [Wagner JE, et al., Cell stem cell 18(1): 144-155 (2016)] lasting up to 15 days gave a median CD34 expansion of 330-fold, whereas another competing technology involving nicotinamide (NAM) [Horwitz ME, et al., J Clin Invest 124(7): 3121-3128 (2014)] could only increase CD34 cells by 72-fold over 21 days. This indicates that IM-29 was highly potent at expanding CD34 selected grafts, attaining significantly better expansion in a shorter period of time. This could save both cost of reagents (less media, cytokine and small molecule replenishment compared to SR1 and NAM) and the duration needed to produce such cellular therapy products. Several clinical trials have attempted to overcome the problem of low cell dose and slow hematopoietic recovery associated with UCBT using the following two broad methods summarized in Tables 1 and 2, together with their major pitfall/s:

**Table 1. Increasing absolute number of infused total nucleated cells:**

| **Approach/es** | **Shortcoming/s** |
|---|---|
| **(i)** Dual unit UCBT (dUCBT) [Sideri A, et al. Haematologica 96(8): 1213-1220 (2011)]; and | **(i)** Complex three-way HLA matching; |
| | **(ii)** higher incidence of GVHD that increases possibility of transplant related mortality (TRM). |
| **(ii)** single unit UCBT combined with haplo-identical CD34 cells (UCB+Haplo CD34) [Liu H, et al., Blood 118(24): 6438-6445 (2011)]. | |
| ***Ex vivo*** expansion of a single unit of UCB which was co-transplanted with an unmanipulated unit. To date, clinical expansion has been done using **(i)** various cytokine cocktails [Shpall EJ, et al., Biol Blood Marrow Transplant 8(7): 368-376 (2002)]; (ii) bioreactors [Jaroscak J, et al. Blood 101(12): 5061-5067 (2003)]; (iii) co-culture with mesenchymal stromal cells (MSC) [de Lima M, et al., N Engl J Med 367(24): 2305-2315 (2012)]; and exogenous addition of (iv) biomolecules such as notch [Delaney C, et al., Nat Med 16(2): 232-236 (2010)]; and **(v)** chemical/small molecules which includes - nicotinamide (NAM - SIRT1 inhibitor) [Horwitz ME, et al., J Clin Invest 124(7): 3121-3128 (2014)]; stemregenin 1 (SR1 - antagonist of aryl hydrocarbon receptor) [Wagner JE, et al., Cell stem cell 18(1): 144-155 (2016)]; and tetraethylenepentamine (TEPA - copper chelator) [de Lima M, et al., Bone Marrow Transplant 41(9): 771-778 (2008)]. | **(i)** Prior stem cell selection was a prerequisite for successful expansion in all protocols except MSC co-culture. |
| | (ii) All protocols required about 3 - 7 growth factors. |
| | (iii) Expanded cells gave early engraftment; sought after long-term hematopoiesis was only conferred by the unmanipulated units in all protocols except NAM and SR1; the latter achieved success by "adding back" the unmanipulated T-cell fraction of the expanded unit during infusion. |

**Table 2. Improving homing of infused/transplanted cells:**

| **Approach/es** | **Shortcoming/s** |
|---|---|
| **(i)** Intrabone marrow infusion (i.b. infusion) of singe UCB unit with or without intravenous (i.v.) infusion of another unmanipulated unit [Hägglund H, et al., Bone Marrow Transplant 21(4): 331-335 (1998)]; and | **(i)** Complex and invasive transplantation protocol that did not result in any clinical benefits with regard to engraftment, blood count recovery and mortality. |
| **(ii)** i.v. co-administration of single UCB along with MSC (UCB+MSC) [Macmillan ML, et al., Bone Marrow Transplant 43(6): 447-54 (2009)]. | |
| **Priming** of an UCB unit with various chemicals and bio-molecules such as **(i)** dimethyl-prostaglandin E2 (dmPGE2) [Cutler C, et al., Blood 122(17): 3074-3081 (2013)]; **(ii)** complement fragment 3a (C3a) [Brunstein CG, et al., Biol Blood Marrow Transplant 19(10): 1474-1479 (2013)]; and **(iii)** fucosylation in the setting of dual unit UCBT [Popat U, et al., Blood 125(19): 2885-2892 (2015)]. | **(i)** In the C3a, fucosylation and cohort 1 of the dm-PGE2 studies, the majority of the patients achieved long-term hematopoiesis from the larger cell dosed non-manipulated unit, thus showing a marginal benefit of manipulating the smaller cell dosed unit. |
| | **(ii)** In cohort 2 of the dm-PGE2 study, the higher |
| | TNC-containing UCB unit was manipulated while the smaller graft was infused without any manipulation. Long-term hematopoiesis was contributed by the manipulated graft, which is to |
| | be expected since grafts with higher cell dose usually dominate engraftment in dUCBT. Such outcomes raise concerns on the efficacy of dm-PGE2 priming. |

Most of the UCB manipulation attempts described in Tables 1 and 2, above, have failed to concurrently address the problem of limited cell dosage, quick neutrophil and platelet recovery (<14 days post-transplant) along with lasting hematopoiesis using only one UCB graft. To date, *ex vivo* expansion has proved to be the most promising technology, but in most cases (>60%) it has only resulted in moderately early engraftment, whereas life-long hematopoiesis was contributed by a co-infused unmanipulated unit. Also, all the above expansion protocols require prior enrichment of stem cells using cell surface markers against CD34 or CD133. The time to neutrophil recovery (defined by absolute neutrophil count of > 500 cells *per* µl of blood for three consecutive days), which is an early measure of transplant success, for the above mentioned current approaches, together with the conditioning regimen that was used, is summarized in Figure 17 with concurrent comparison to conventional HSCT transplants. However, we show that expansion using an azole-based small molecule allows an individual graft to have sufficient cell dose which the non-expanded counterpart would not possess.

### SUMMARY

Fresh human umbilical cord blood (UCB) was the source of hematopoietic stem and progenitor cells (HSPC) in the present study.

UCB mononucleated cells (UCB-MNC) were obtained from fresh samples by performing density dependent centrifugation (Figure 3). For expansion, these MNC do not need to be enriched for CD34 expression using magnetic selection. However, samples enriched for CD34+ cells are also suitable for expansion using azole-based small molecules according to the invention.

Since, in the clinical setting, only frozen samples are available for either expansion or transplantation, the UCB-MNC were frozen down before being thawed out for further experimentation (Figure 3).

The UCB MNC fraction comprises red blood cells (RBC) that do not express CD45, and white blood cells (WBC) that express CD45. HSPC is a subset of the nucleated WBC and express the antigen CD34 together with CD45 (Figure 4).

HSPC is classified into different subsets by expression of different antigens (Figure 4):
a. Hematopoietic progenitor cells (HPC) → CD45+CD34+CD38-CD45RA- (highest frequency but minimal self-renewal capacity)
b. Hematopoietic stem cells 1 (HSC1) -7 CD45+CD34+CD38-CD45RA-CD90+ (moderate frequency and self-renewal capacity)
c. Hematopoietic stem cells 2 (HSC2) -> CD45+CD34+CD38-CD45RA-CD90+CD49f+ (lowest frequency but highest self-renewal capacity)
   IM-29 was the most effective compound for expanding HSPC. The structure of IM-29 is shown in Figure 6(A).
   IM-04 was the second most effective compound for expanding HSPC. The structure of IM-04 is shown in Figure 6(B).

The working concentration of IM-29 and other structural analogues is 5.0 µM.

The cell population preferred for initiating expansion cultures with IM-29 is UCB mononucleated cells i.e. no prior stem cell selection using cell surface markers such as CD34 and CD133 is required to achieve sufficient expansion.

We have shown that serum-free expansion media (StemSpan^{™}-SFEM) and animal-component-free (StemSpan^{™}-ACF) media could be used for expanding UCB MNC in the presence of IM-29 (Figure 10). Other stem cell expansion media may also be suitable.

A cytokine cocktail was added to all expansion cultures (with or without IM-29) and comprised 100 ng/ml of stem cell factor (SCF) and thrombopoietin (TPO); 50 ng/ml of Fms-related tyrosine kinase 3 ligand (FLT-3L); and 20 ng/ml of insulin-like growth factor binding protein 2 (IGFBP-2) (Figures 1 and 2).

The physical conditions used in the Examples for expanding a UCB graft in the presence of IM-29 includes a temperature of 37°C with 5% CO₂ (Figure 2). However, it is known that hematopoietic stem and progenitor cells may be cultured in hypoxic incubators to better mimic the natural stem cell niche of the bone marrow microenvironment. It is likely that the present invention will also work in hypoxic culturing conditions.

IM-29 and all the structural analogues had minimal toxicity on UCB cells by day 3 (Figure 7).

An expansion culture for UCB MNC with IM-29 lasts for about 7 to 11 days. An optimal expansion culture duration was found to be 10 days as measured by phenotypic assay (Figure 11).

IM-29 is preferably added at the point of initiating culturing and also at day 7 when media and cytokines are replenished for optimal expansion (Figure 12).

HSPC that express CD90 (HSC1) and CD49f (HSC2) are expanded when cultures are initiated with UCB MNC (Figure 13). The expanded cells do not exhibit cytogenetic abnormalities or leukemic transformation (Figure 13).

IM-29 expanded grafts (fresh or frozen-thawed) could repopulate NSG mice blood as early as week 2-3 (primary engraftment of CD34 progenitor and myeloid cells) and the engraftment lasted until at least week 19-20 in the bone marrow (multi-lineage reconstitution of human cells compromising of stem and progenitor cells, myeloid and lymphoid cells) (Figures 15-17).

IM-29 mediated expansion of UCB overcomes the following problems associated with UCB being used as a graft for allergenic transplantation in adults:
1. Overcomes low cell dose of the graft since it increases the total nucleated cells by at least 5-fold.
2. Expands hematopoietic stem and progenitor cells. Specifically it expands hematopoietic progenitor cells (HPC: CD45+CD34+CD38-CD45RA-) by at least 1,000-fold. Expansion at such scale has not been reported before using just a small molecule. In all other established protocols such expansion scale was achieved only when cultures were initiated with selected/purified CD34/CD133 cells. Also, to the best of our knowledge, this is the first expansion protocol that reports the expansion of rare HSPC cells that are defined by phenotypic expression of (a) CD45+CD34+CD38-CD45RA-CD90+ (HSC1); and (b) CD45+CD34+CD38-CD45RA-CD90+CD49f+ (HSC2).
3. The expanded UCB graft maintains stem and progenitor cells' functionality as determined using *in vitro* and *in vivo* functional assays. Specifically, transplantation of the IM-29 expanded graft to sub-lethally irradiated immunodeficient mice results in faster engraftment of human cells as shown by chimerism in peripheral blood by week 3. Until now, obtaining fast blood count recovery (< 3 weeks) from expanded graft has been a challenge in both xenotransplantation studies and human clinical trials. Finally, the grafts showed the ability to sustain long term multi-lineage hematopoiesis since they could be detected in the bone marrow of the recipient immunodeficient mice after 19-20 weeks of transplantation.

In the IM-29 mediated expansion protocol, only one unit of UCB is required to give rise to a sufficient number of stem and progenitor cells (>25 million cells/kg) that have the following advantages compared to current approaches:
1. To obtain clinically relevant expansion of HSPC it is not necessary to perform a prior stem cell selection; nor is supplementation of fetal bovine serum in culture media necessary. From the clinical perspective, by-passing pre-selection of cells is an advantage since it eliminates the need for an additional manipulative step that could result in a loss of very primitive stem/progenitor cells, especially those that do not express the surface markers required by the selection methods.
2. Most expansion technologies require a complex cytokine cocktail, of which some are late acting cytokines that rapidly promote differentiation at the expense of self-renewal. However, the proposed approach uses a simple cocktail of four growth factors together with a small molecule to achieve expansion, thus simplifying procedures.
3. Only a single unit of UCBT is required to obtain an IM-29-expanded graft, which reduces the HLA matching complexity compared to current clinical practice where two unmanipulated units are transplanted simultaneously to achieve sufficient cell dose, albeit at a higher incidence of graft-versus-host-disease.

### BIBLIOGRAPHY

Ballen KK, Gluckman E, Broxmeyer HE. Umbilical cord blood transplantation: the first 25 years and beyond. Blood 2013; 122(4): 491-498.
Bari S, Seah KKH, Poon Z, Cheung AMS, Fan X, Ong SY, et al. Expansion and Homing of Umbilical Cord Blood Hematopoietic Stem and Progenitor Cells for Clinical Transplantation. Biol Blood Marrow Transplant 2015; 21(6): 1008-19.
Barker JN, Byam CE, Kernan NA, Lee SS, Hawke RM, Doshi KA, et al. Availability of cord blood extends allogeneic hematopoietic stem cell transplant access to racial and ethnic minorities. Biol Blood Marrow Transplant 2010; 16(11): 1541-1548.
Brunstein CG, McKenna DH, DeFor TE, Sumstad D, Paul P, Weisdorf DJ, et al. Complement fragment 3a priming of umbilical cord blood progenitors: safety profile. Biol Blood Marrow Transplant 2013; 19(10): 1474-1479.
Cunha R, Loiseau P, Ruggeri A, Sanz G, Michel G, Paolaiori A, et al. Impact of HLA mismatch direction on outcomes after umbilical cord blood transplantation for hematological malignant disorders: a retrospective Eurocord-EBMT analysis. Bone Marrow Transplant 2014; 49(1): 24-29.
Cutler C, Multani P, Robbins D, Kim HT, Le T, Hoggatt J, et al. Prostaglandin-modulated umbilical cord blood hematopoietic stem cell transplantation. Blood 2013; 122(17): 3074-3081.
Dahlberg A, Delaney C, Bernstein ID. Ex vivo expansion of human hematopoietic stem and progenitor cells. Blood 2011; 117(23): 6083-6090.
Delaney C, Heimfeld S, Brashem-Stein C, Voorhies H, Manger RL, Bernstein ID, et al. Notch-mediated expansion of human cord blood progenitor cells capable of rapid myeloid reconstitution. Nat Med 2010; 16(2): 232-236.
de Lima M, McMannis J, Gee A, Komanduri K, Couriel D, Andersson BS, et al. Transplantation of ex vivo expanded cord blood cells using the copper chelator tetraethylenepentamine: a phase I/II clinical trial. Bone Marrow Transplant 2008; 41(9): 771-778.
de Lima M, McNiece I, Robinson SN, Munsell M, Eapen M, Horowitz M, et al. Cord-blood engraftment with ex vivo mesenchymal-cell coculture. N Engl J Med 2012; 367(24): 2305-2315.
Fan Xiubo et al. Low-dose insulin-like growth factor binding proteins 1 and 2 and angiopoietin-like protein 3 coordinately stimulate ex vivo expansion of human umbilical cord blood hematopoietic stem cells as assayed in NOD/SCIO gamma null mice. Stem Cell Research & Therapy 2014; 5(3): 71.
Gluckman E, Devergié A, Bourdeau-Esperou H, Thierry D, Traineau R, Auerbach A, et al. Transplantation of umbilical cord blood in Fanconi's anemia. Nouv Rev Fr Hematol 1990; 32(6): 423-425.
Gluckman E, Rocha V. History of the clinical use of umbilical cord blood hematopoietic cells. Cytotherapy 2005; 7(3): 219-227.
Gratwohl A, Baldomero H, Aljurf M, Pasquini MC, Bouzas LF, Yoshimi A, et al. Hematopoietic stem cell transplantation: a global perspective. JAMA 2010; 303(16): 1617-1624.
Hägglund H, Ringdén O, Agren B, Wennberg L, Remberger M, Rundquist L, et al. Intraosseous compared to intravenous infusion of allogeneic bone marrow. Bone Marrow Transplant 1998; 21(4): 331-335.
Hofmeister CC, Zhang J, Knight KL, Le P, Stiff PJ. Ex vivo expansion of umbilical cord blood stem cells for transplantation: growing knowledge from the hematopoietic niche. Bone Marrow Transplant 2007; 39(1): 11-23.
Horwitz ME, Chao NJ, Rizzieri DA, Long GD, Sullivan KM, Gasparetto C, et al. Umbilical cord blood expansion with nicotinamide provides long-term multilineage engraftment. J Clin Invest 2014; 124(7): 3121-3128.
Hwang WYK, Samuel M, Tan D, Koh LP, Lim W, Linn YC, et al. A meta-analysis of unrelated donor umbilical cord blood transplantation versus unrelated donor bone marrow transplantation in adult and pediatric patients. Biol Blood Marrow Transplant 2007; 13(4): 444-453.
Jaroscak J, Goltry K, Smith A, Waters-Pick B, Martin PL, Driscoll TA, et al. Augmentation of umbilical cord blood (UCB) transplantation with ex vivo-expanded UCB cells: results of a phase 1 trial using the AastromReplicell System. Blood 2003; 101(12): 5061-5067.
Kelly SS, Sola CBS, de Lima M, Shpall E. Ex vivo expansion of cord blood. Bone Marrow Transplant 2009; 44(10): 673-681.
Komanduri KV, St John LS, de Lima M, McMannis J, Rosinski S, McNiece I, et al. Delayed immune reconstitution after cord blood transplantation is characterized by impaired thymopoiesis and late memory T-cell skewing. Blood 2007; 110(13): 4543-4551.
Liu H, Rich ES, Godley L, Odenike O, Joseph L, Marino S, et al. Reduced-intensity conditioning with combined haploidentical and cord blood transplantation results in rapid engraftment, low GVHD, and durable remissions. Blood 2011; 118(24): 6438-6445.
Lund TC, Boitano AE, Delaney CS, Shpall EJ, Wagner JE. Advances in umbilical cord blood manipulation-from niche to bedside. Nature reviews. Clinical oncology 2015; 12(3): 163-74.
Popat U, Mehta RS, Rezvani K, Fox P, Kondo K, Marin D, et al. Enforced fucosylation of cord blood hematopoietic cells accelerates neutrophil and platelet engraftment after transplantation. Blood 2015; 125(19): 2885-2892.
Macmillan ML, Blazar BR, DeFor TE, Wagner JE. Transplantation of ex-vivo culture-expanded parental haploidentical mesenchymal stem cells to promote engraftment in pediatric recipients of unrelated donor umbilical cord blood: results of a phase I-II clinical trial. Bone Marrow Transplant. 2009; 43(6): 447-54.
McDermott SP, Eppert K, Lechman ER, Doedens M, Dick JE. Comparison of human cord blood engraftment between immunocompromised mouse strains. Blood. 2010; 116(2): 193-200.
Notta F, Doulatov S, Dick JE. Engraftment of human hematopoietic stem cells is more efficient in female NOD/SCID/IL-2Rgc-null recipients. Blood. 2010; 115(18): 3704-7.
Shpall EJ, Quinones R, Giller R, Zeng C, Baron AE, Jones RB, et al. Transplantation of ex vivo expanded cord blood. Biol Blood Marrow Transplant 2002; 8(7): 368-376.
Sideri A, Neokleous N, Brunet De La Grange P, Guerton B, Le Bousse Kerdilles MC, Uzan G, et al. An overview of the progress on double umbilical cord blood transplantation. Haematologica 2011; 96(8): 1213-1220.
Voelker R. FDA grants approval for first cord blood product. JAMA 2011; 306(22): 2442.
Wagner JE, Brunstein CG, Boitano AE, DeFor TE, McKenna D, Sumstad D, et al. Phase I/II Trial of StemRegenin-1 Expanded Umbilical Cord Blood Hematopoietic Stem Cells Supports Testing as a Stand-Alone Graft. Cell stem cell 2016; 18(1): 144-155.

## Claims

1. A method for ex *vivo* expansion of a total nucleated cells and/or a subset of a CD45+CD34+ hematopoietic stem cells and progenitor cells component of an umbilical cord blood, bone marrow or mobilized peripheral blood sample comprising the steps of:
(i) culturing a total nucleated cells or a mononucleated cell fraction or CD45+CD34+ hematopoietic stem cells and progenitor cells component of the sample in media; and
(ii) contacting the cell(s) of step (i) with a composition comprising at least one azole-based small molecule,
wherein the at least one azole based small molecule is represented by formula (I), 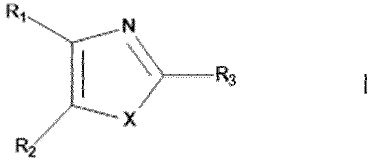 wherein:
X represents NR₄ or O, where R₄ is H or methyl;
R₁ represents phenyl or pyridinyl (which are unsubstituted or substituted with one or more substituents selected from CI, Br, F and methyl, (which latter group is unsubstituted or substituted with F));
R₂ represents phenyl, pyridyl or dihydropyranyl (which are unsubstituted or substituted with one or more substituents selected from CI, Br, F and methyl (which latter group is unsubstituted or substituted with F));
R₃ represents H, or naphthyl which is unsubstituted or substituted with one or more groups selected from CI, F and OR₅ where R₅ is H or methyl, or
salts and solvates thereof.

2. The method of claim 1, wherein the at least one azole-based small molecule is selected from:
(A) the list:
(i) 4-[2-(1-fluoronaphthalen-2-yl)-4(5)-(4-fluorophenyl)-1*H*-imidazol-5(4)-yl]pyridine;
(ii) 4-[2-(1-fluoronaphthalen-2-yl)-4-(*m*-tolyl)-1*H*-imidazol-5-yl]pyridine;
(iii) 4-[2-(naphthalen-2-yl)-4(5)-(*m*-tolyl)-1H-imidazol-5(4)-yl]pyridine;
(iv) 4-[2-(naphthalen-2-yl)-4(5)-(4-fluorophenyl)-1*H*-imidazol-5(4)-yl]pyridine;
(v) 4-[2-(1-bromonaphthalen-2-yl)-4(5)-(4-fluorophenyl)-1*H*-imidazol-5(4)-yl]pyridine;
(vi) 4-[2-(1-fluoronaphthalen-2-yl)-4-[3-(trifluoromethyl)phenyl]-1*H*-imidazol-5-yl]pyridine;
(vii) 2-(1-fluoronaphthalen-2-yl)-4-(pyridin-4-yl)-5-(*m*-tolyl)oxazole;
(viii) 5(4)-(3,6-dihydro-2*H*-pyran-4-yl)-2-(1-fluoronaphthalen-2-yl)-4(5)-(*m*-tolyl)-1*H-*imidazole;
(ix) 5(4)-(3,6-dihydro-2*H*-pyran-4-yl)-2-(6-methoxynaphthalen-2-yl)-4(5)-(*m*-tolyl)-1*H*-imidazole; and
(x) 5(4)-(3,6-dihydro-2*H*-pyran-4-yl)-2-(1-fluoronaphthalen-2-yl)-4(5)-(4-fluorophenyl)-1*H*-imidazole;
(xi) 4-(4(5)-(4-fluorophenyl)-2-(7-methoxynaphthalen-2-yl)-1*H*-imidazol-5(4)-yl)pyridine;
(xii) 4-[4(5)-(*m*-tolyl)-1*H*-imidazol-5(4)-yl]pyridine; and
(xiii) 4-[4(5)-(4-fluorophenyl)-1H-imidazol-5(4)-yl]pyridine; or
(B) the list:
(i) 4-[2-(1-fluoronaphthalen-2-yl)-4(5)-(4-fluorophenyl)-1*H*-imidazol-5(4)-yl]pyridine;
(ii) 4-[2-(1-fluoronaphthalen-2-yl)-4-(m-tolyl)-1*H*-imidazol-5-yl]pyridine;
(iii) 4-[2-(naphthalen-2-yl)-4(5)-(*m*-tolyl)-1H-imidazol-5(4)-yl]pyridine;
(iv) 4-[2-(naphthalen-2-yl)-4(5)-(4-fluorophenyl)-1*H*-imidazol-5(4)-yl]pyridine;
(v) 4-[2-(1-bromonaphthalen-2-yl)-4(5)-(4-fluorophenyl)-1*H*-imidazol-5(4)-yl]pyridine;
(vi) 4-[2-(1-fluoronaphthalen-2-yl)-4-[3-(trifluoromethyl)phenyl]-1*H*-imidazol-5-yl]pyridine; and
(vii) 2-(1-fluoronaphthalen-2-yl)-4-(pyridin-4-yl)-5-(*m*-tolyl)oxazole.

3. The method of claim 1 or 2, wherein the hematopoietic stem cells and progenitor cells are expanded in the presence of:
(a) at least one cytokine selected from the group comprising stem cell factor (SCF), thrombopoietin (TPO), Fms-related tyrosine kinase 3 ligand (FLT-3L), interleukin 3 (IL-3), interleukin 6 (IL-6), granulocyte-colony stimulating factor (GCSF) and insulin-like growth factor binding protein 2 (IGFBP-2); or
(b) SCF, TPO, FLT-3L and IGFBP-2.

4. The method of any one of the preceding claims, comprising culturing the umbilical cord blood, bone marrow and/or mobilized peripheral blood mononuclear cell(s) with the at least one azole-based small molecule for;
i) a period of at least 9 days; or
ii) a period of about 11 days.

5. The method of claim 3 or 4, wherein the cytokines are added to the culture at day 0 and/or at day 7 and/or the at least one azole-based small molecule is added to the culture at day 0 and/or at day 7.

6. The method according to any one of the preceding claims, further comprising the step of harvesting the cells after about 10 to 11 days in culture.

7. The method according to any one of the preceding claims, further comprising the step of separately retaining a CD34- cell fraction (comprising lymphoid cells) for later co-transplantation with the *ex vivo* expanded cells.

8. A composition comprising at least one azole-based small molecule defined in claim 1 or 2; and at least one cytokine selected from the group comprising SCF, TPO, FLT-3L and IGFBP-2 for use in *ex vivo* expansion of the hematopoietic stem cells and progenitor cells component of umbilical cord blood, bone marrow and/or mobilized peripheral blood.

9. Use of a composition comprising at least one azole-based small molecule defined in claims 1 or 2 in *ex vivo* expansion of the hematopoietic stem cells and progenitor cells component of umbilical cord blood, bone marrow and/or mobilized peripheral blood.

## Patentansprüche

1. Verfahren zur *Ex-vivo*-Expansion einer Komponente von gesamten kernhaltigen Zellen und/oder einer Teilmenge aus einer CD45+CD34+ Hämatopoetische-Stammzellen- und -Vorläuferzellen-Komponente einer Probe von Nabelschnurblut, Knochenmark oder mobilisiertem peripherem Blut, umfassend die Schritte:
(i) Kultivieren einer Komponente von gesamten kernhaltigen Zellen oder einer Fraktion einer einkerniger Zelle oder einer CD45+CD34+ Hämatopoetische-Stammzellen- und -Vorläuferzellen-Komponente der Probe in Medien; und
(ii) In-Kontakt-Bringen der Zelle/-n aus Schritt (i) mit einer Zusammensetzung, die mindestens ein kleines Molekül auf Azolbasis umfasst,
wobei das mindestens eine kleine Molekül auf Azolbasis dargestellt ist durch Formel (I), 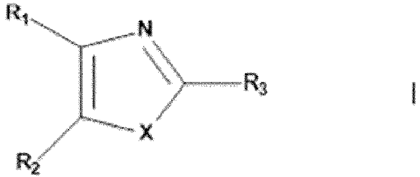 wobei:
X NR₄ oder O darstellt, worin R₄ H oder Methyl ist;
R₁ Phenyl oder Pyridinyl darstellt (die unsubstituiert sind oder mit einem oder mehreren Substituenten substituiert sind, ausgewählt aus Cl, Br, F und Methyl (welche letztere Gruppe unsubstituiert ist oder mit F substituiert ist));
R₂ Phenyl, Pyridyl oder Dihydropyranyl darstellt (die unsubstituiert sind oder mit einem oder mehreren Substituenten subsituiert sind, ausgewählt aus Cl, Br, F und Methyl (welche letztere Gruppe unsubstituiert ist oder mit F substituiert ist));
R₃ H oder Naphthyl darstellt, das unsubstituiert ist oder mit einer oder mehreren Gruppen substituiert ist, ausgewählt aus Cl, F und OR₅, worin R₅ H oder Methyl ist, oder
Salze und Solvate davon.

2. Verfahren nach Anspruch 1, wobei das mindestens eine kleine Molekül auf Azolbasis ausgewählt ist aus:
(A) der Liste:
(i) 4-[2-(1-Fluornaphthalin-2-yl)-4(5)-(4-fluorphenyl)-1*H*-imidazol-5(4)-yl]pyridin;
(ii) 4-[2-(1-Fluornaphthalin-2-yl)-4-(*m*-tolyl)-1*H*-imidazol-5-yl]pyridin;
(iii) 4-[2-(Naphthalin-2-yl)-4(5)-(*m*-tolyl)-1*H*-imidazol-5(4)-yl]pyridin;
(iv) 4-[2-(Naphthalin-2-yl)-4(5)-(4-fluorphenyl)-1*H*-imidazol-5(4)-yl]pyridin;
(v) 4-[2-(1-Bromnaphthalin-2-yl)-4(5)-(4-fluorphenyl)-1*H*-imidazol-5(4)-yl]pyridin;
(vi) 4-[2-(1-Fluomaphthalin-2-yl)-4-[3-(trifluormethyl)phenyl]-1*H*-imidazol-5-yl]pyridin;
(vii) 2-(1-Fluornaphthalin-2-yl)-4-(pyridin-4-yl)-5-(*m*-tolyl)oxazol;
(viii) 5(4)-(3,6-Dihydro-2*H*-pyran-4-yl)-2-(1-fluornaphthalin-2-yl)-4(5)-(*m-*tolyl)-1*H*-imidazol;
(ix) 5(4)-(3,6-Dihydro-2*H*-pyran-4-yl)-2-(6-methoxynaphthalin-2-yl)-4(5)-(*m-*tolyl)-1*H*-imidazol; und
(x) 5(4)-(3,6-Dihydro-2*H*-pyran-4-yl)-2-(1-fluornaphthalin-2-yl)-4(5)-(4-fluorphenyl)-1*H*-imidazol;
(xi) 4-(4(5)-(4-Fluorphenyl)-2-(7-methoxynaphthalin-2-yl)-1*H*-imidazol-5(4)-yl)pyridin;
(xii) 4-[4(5)-(*m*-Tolyl)-1*H*-imidazol-5(4)-yl]pyridin; und
(xiii) 4-[4(5)-(4-Fluorphenyl)-1*H*-imidazol-5(4)-yl]pyridin; oder
(B) der Liste:
(i) 4-[2-(1-Fluornaphthalin-2-yl)-4(5)-(4-fluorphenyl)-1*H*-imidazol-5(4)-yl]pyridin;
(ii) 4-[2-(1-Fluornaphthalin-2-yl)-4-(*m*-tolyl)-1*H*-imidazol-5-yl]pyridin;
(iii) 4-[2-(Naphthalin-2-yl)-4(5)-(*m*-tolyl)-1*H*-imidazol-5(4)-yl]pyridin;
(iv) 4-[2-(Naphthalin-2-yl)-4(5)-(4-fluorphenyl)-1*H*-imidazol-5(4)-yl]pyridin;
(v) 4-[2-(1-Bromnaphthalin-2-yl)-4(5)-(4-fluorphenyl)-1*H*-imidazol-5(4)-yl]pyridin;
(vi) 4-[2-(1-Fluornaphthalin-2-yl)-4-[3-(trifluormethyl)phenyl]-1*H*-imidazol-5-yl]pyridin; und
(vii) 2-(1-Fluornaphthalin-2-yl)-4-(pyridin-4-yl)-5-(*m*-tolyl)oxazol.

3. Verfahren nach Anspruch 1 oder 2, wobei die hämatopoetischen Stammzellen und Vorläuferzellen expandiert werden in Gegenwart von:
(a) mindestens einem Zytokin, ausgewählt aus der Gruppe, umfassend Stammzellfaktor (SCF), Thrombopoietin (TPO), Fms-verwandte-Tyrosinkinase-3-Ligand (FLT-3L), Interleukin-3 (IL-3), Interleukin-6 (IL-6), Granulozyten-Koloniestimulierenden Faktor (GCSF) und insulinähnlichen Wachstumsfaktor bindendes Protein-2 (IGFBP-2); oder
(b) SCF, TPO, FLT-3L und IGFBP-2.

4. Verfahren nach einem der vorstehenden Ansprüche, umfassend Kultivieren der mononukleären Zelle/-n des Nabelschnurbluts, Knochenmarks und/oder mobilisierten peripheren Bluts mit dem mindestens einen kleinen Molekül auf Azolbasis für:
i) einen Zeitraum von mindestens 9 Tagen; oder
ii) einen Zeitraum von ungefähr 11 Tagen.

5. Verfahren nach Anspruch 3 oder 4, wobei der Kultur die Zytokine an Tag 0 und/oder an Tag 7 zugegeben werden und/oder der Kultur das mindestens eine kleine Molekül auf Azolbasis an Tag 0 und/oder an Tag 7 zugegeben wird.

6. Verfahren nach einem der vorstehenden Ansprüche, ferner umfassend den Schritt des Erntens der Zellen nach ungefähr 10 bis 11 Tagen in Kultur.

7. Verfahren nach einem der vorstehenden Ansprüche, ferner umfassend den Schritt des gesonderten Zurückbehaltens einer CD34- Zellfraktion (welche lymphoide Zellen umfasst) zwecks späterer Ko-Transplantation mit den *ex vivo* expandierten Zellen.

8. Zusammensetzung, umfassend mindestens ein in Anspruch 1 oder 2 definiertes kleines Molekül auf Azolbasis, und mindestens ein Zytokin, ausgewählt aus der SCF, TPO, FLT-3L und IGFBP-2 umfassenden Gruppe, zur Verwendung in der *Ex-vivo*-Expansion der Hämatopoetische-Stammzellen- und -Vorläuferzellen-Komponente von Nabelschnurblut, Knochenmark und/oder mobilisiertem peripherem Blut.

9. Verwendung einer Zusammensetzung, umfassend mindestens ein in Anspruch 1 oder 2 definiertes kleines Molekül auf Azolbasis, in der *Ex-vivo*-Expansion der Hämatopoetische-Stammzellen- und -Vorläuferzellen-Komponente von Nabelschnurblut, Knochenmark und/oder mobilisiertem peripherem Blut.

## Revendications

1. Procédé d'expansion *ex vivo* d'une composante de cellules nucléées totales et/ou d'un sous-ensemble d'une composante de cellules souches et de cellules progénitrices hématopoïétiques CD45+CD34+ d'un échantillon de sang de cordon ombilical, de moelle osseuse ou de sang périphérique mobilisé comprenant les étapes suivantes :
(i) la culture d'une composante de cellules nucléées totales ou d'une fraction de cellule mononucléée ou d'une composante de cellules souches et progénitrices hématopoïétiques CD45+CD34+ de l'échantillon dans des milieux, et
(ii) la mise en contact de(s) (la) cellule(s) de l'étape (a) avec une composition comprenant au moins une petite molécule à base d'azole,
dans lequel l'au moins une petite molécule à base d'azole est représentée par la formule (I) : 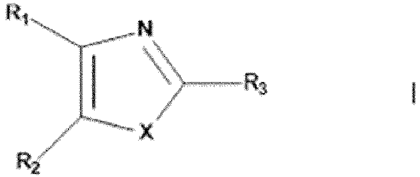 dans laquelle :
X représente NR₄ ou O, R₄ étant H ou méthyl,
R₁ représente phényl ou pyridinyl (qui sont non substitués ou substitués par un ou plusieurs substituants choisis parmi Cl, Br, F et méthyl (ce dernier groupe étant non substitué ou substitué par F)),
R₂ représente phényl, pyridyl ou dihydropyranyl (qui sont non substitués ou substitués par un ou plusieurs substituants choisis parmi Cl, Br, F et méthyl (ce dernier groupe étant non substitué ou substitué par F)),
R₃ représente H, ou naphtyl qui est non substitué ou substitué par un ou plusieurs substituants choisis parmi Cl, F et OR₅, où R₅ est H ou méthyl, ou
des sels et des solvates de celle-ci.

2. Procédé selon la revendication 1, dans lequel l'au moins une petite molécule à base d'azole est sélectionnée parmi :
(A) la liste suivante :
(i) 4-[2-(1-fluoronaphtalén-2-yl)-4(5)-(4-fluorophényl)-1*H*-imidazol-5(4)-yl]pyridine,
(ii) 4-[2-(1-fluoronaphtalén-2-yl)-4-(*m*-tolyl)-1*H*-imidazol-5-yl]pyridine,
(iii) 4-[2-(naphtalén-2-yl)-4(5)-(*m*-tolyl)-1*H*-imidazol-5(4)-yl]pyridine,
(iv) 4-[2-(naphtalén-2-yl)-4(5)-(4-fluorophényl)-1*H*-imidazol-5(4)-yl]pyridine,
(v) 4-[2-(1-bromonaphtalén-2-yl)-4(5)-(4-fluorophényl)-1*H*-imidazol-5(4)-yl]pyridine,
(vi) 4-[2-(1-fluoronaphtalén-2-yl)-4-[3-(trifluorométhyl)phényl)]-1*H*-imidazol-5-yl]pyridine,
(vii) 2-(1-fluoronaphtalén-2-yl)-4-(pyridyn-4-yl)-5-(*m*-tolyl)oxazole,
(viii) 5(4)-(3,6-dihydro-2*H*-pyran-4-yl)-2-(1-fluoronaphtalén-2-yl)-4(5)-(*m-*tolyl)-1*H*-imidazole,
(ix) 5(4)-(3,6-dihydro-2*H*-pyran-4-yl)-2-(6-méthoxynaphtalén-2-yl)-4(5)-(*m-*tolyl)-1*H*-imidazole, et
(x) 5(4)-(3,6-dihydro-2*H*-pyran-4-yl)-2-(1-fluoronaphtalén-2-yl)-4(5)-(4-fluorophényl)-1*H*-imidazole,
(xi) 4-(4(5)-(4-fluorophényl)-2-(7-méthoxynaphtalén-2-yl)-1*H*-imidazol-5(4)-yl)pyridine,
(xii) 4-[4(5)-(*m*(tolyl)-1*H*-imidazol-5(4)-yl]pyridine, et
(xiii) 4-[4(5)-(4-fluorophényl)-1*H*-imidazol-5(4)-yl]pyridine, ou
(B) la liste suivante :
(i) 4-[2-(1-fluoronaphtalén-2-yl)-4(5)-(4-fluorophényl)-1*H*-imidazol-5(4)-yl]pyridine,
(ii) 4-[2-(1-fluoronaphtalén-2-yl)-4-(*m*-tolyl)-1*H*-imidazol-5-yl]pyridine,
(iii) 4-[2-(naphtalén-2-yl)-4(5)-(*m*-tolyl)-1*H*-imidazol-5(4)-yl]pyridine,
(iv) 4-[2-(naphtalén-2-yl)-4(5)-(4-fluorophényl)-1*H*-imidazol-5(4)-yl]pyridine,
(v) 4-[2-(1-bromonaphtalén-2-yl)-4(5)-(4-fluorophényl)-1*H*-imidazol-5(4)-yl]pyridine,
(vi) 4-[2-(1-fluoronaphtalén-2-yl)-4-[3-(trifluorométhyl)phényl)]-1*H*-imidazol-5-yl]pyridine, et
(vii) 2-(1-fluoronaphtalén-2-yl)-4-(pyridyn-4-yl)-5-(*m*-tolyl)oxazole.

3. Procédé selon la revendication 1 ou 2, dans lequel les cellules souches et les cellules progénitrices hématopoïétiques sont développées en présence :
(a) d'au moins une cytokine choisie parmi le groupe comprenant un facteur des cellules souches (SCF), la thrombopoïétine (TPO), un ligand de tyrosine kinase 3 apparenté à FMS (FLT-3L), l'interleukine 3 (IL-3), l'interleukine 6 (IL-6), un facteur stimulant les colonies de granulocytes (GCSF) et une protéine 2 se liant au facteur de croissance de type insuline (IGFBP-2), ou
(b) de SCF, TPO, FLT-3L et d'IGFBP-2.

4. Procédé selon l'une quelconque des revendications précédentes, comprenant la culture de cellule(s) mononucléaire(s) de sang de cordon ombilical, de moelle osseuse et/ou de sang périphérique mobilisé avec l'au moins une petite molécule à base d'azole pendant :
i) une période d'au moins 9 jours, ou
ii) une période d'au moins 11 jours.

5. Procédé selon la revendication 3 ou 4, dans lequel les cytokines sont ajoutées à la culture au jour 0 et/ou au 7^{ème} jour et/ou l'au moins une petite molécule à base d'azole est ajoutée à la culture au jour 0 et/ou au 7^{ème} jour.

6. Procédé selon l'une quelconque des revendications précédentes, comprenant en outre l'étape de récolte des cellules après environ 10 à 11 jours en culture.

7. Procédé selon l'une quelconque des revendications précédentes, comprenant en outre l'étape de rétention séparée d'une fraction de cellules CD34- (comprenant des cellules lymphoïdes) pour une co-transplantation ultérieure avec les cellules développées *ex vivo.*

8. Composition comprenant au moins une petite molécule à base d'azole définie dans la revendication 1 ou 2, et au moins une cytokine sélectionnée parmi le groupe comprenant SCF, TPO, FLT-3L et IGFBP-2 pour utilisation dans l'expansion *ex vivo* de la composante de cellules souches et de cellules progénitrices hématopoïétiques de sang de cordon ombilical, de moelle osseuse et/ou de sang périphérique mobilisé.

9. Utilisation d'une composition comprenant au moins une petite molécule à base d'azole définie dans les revendications 1 ou 2 dans l'expansion *ex vivo* de la composante de cellules souches et de cellules progénitrices hématopoïétiques de sang de cordon ombilical, de moelle osseuse et/ou de sang périphérique mobilisé.
